# EUROPEAN PATENT SPECIFICATION

(11) **EP 3 039 162 B1**
(45) Date of publication and mention of the grant of the patent: **02.01.2019**
(21) Application number: 14840425.4
(22) Date of filing: 22.08.2014
(51) Int. Cl.: C12P 21/04, C12Q 1/6881, C12Q 1/02, C40B 30/04, C40B 30/06, G01N 33/00, G01N 33/50, G01N 33/566

(54) **DETERMINATION OF IMMUNE CELLS AND OTHER CELLS**
BESTIMMUNG VON IMMUNZELLEN UND ANDEREN ZELLEN
DÉTERMINATION DE CELLULES IMMUNITAIRES ET D'AUTRES CELLULES

(30) Priority: 26.08.2013 US 201361870214 P
(43) Date of publication of application: 06.07.2016
(73) Proprietor: President and Fellows of Harvard College, Cambridge, MA 02138 (US)
(72) Inventor: WEITZ, David, A., Bolton, MA 01740 (US); HEYMAN, John, Somerville, MA 02143 (US); GILBERT, John, R., Brookline, MA 02446 (US)
(74) Representative: Hoffmann Eitle
(86) International application number: PCT/US2014/052271
(87) International publication number: WO 2015/031190

(56) References cited:
- WO-A1-2009/011808
- WO-A2-2012/167142
- US-A1- 2004 241 759
- US-A1- 2006 023 559
- US-A1- 2010 092 955
- US-A1- 2011 275 063
- US-A1- 2012 149 592
- BROUZES ET AL.: 'Droplet microfluidic technology for single- cell high-throughput screening.' PROC NAT ACAD SCI vol. 106, no. 34, 25 August 2009, pages 14195 - 14200, XP055074334
- TUMARKIN ET AL.: 'High-throughput combinatorial cell co-culture using microfluidics.' vol. 3, no. 6, June 2011, pages 653 - 662, XP055320308
- KONRY ET AL.: 'Droplet-based microfluidic platforms for single T cell secretion analysis of IL -10 cytokine.' BIOSENS BIOELECTRON vol. 26, no. 5, 15 January 2011, pages 2707 - 2710, XP027580225

## Description

### FIELD

The present invention generally relates to fluidic droplets, and to techniques for screening or sorting such fluidic droplets. In some embodiments, the fluidic droplets may contain cells such as immune cells, which can be analyzed to determine receptor sequences or other useful properties of the cells.

### BACKGROUND

Some cellular phenotypes are clonal, meaning that single progenitor cells can give rise to many identical daughter cells, each having the same phenotype. Often, a clonal phenotype is governed by a particular genotype. For example, T-cells present T-Cell Receptor (TCR) molecules on their cell membranes. Each T-cell clone encodes T-Cell Receptor (TCR) molecules of a single sequence. If a T-cell expresses a TCR that binds to peptides presented by infected or cancerous cells, the T-cell can be signaled to divide rapidly. This "clonal expansion" of the original T-cell results in production of a large number of phenotypically identical cells, each encoding the same TCR. During an immune response, these clonally-derived T-cells can be activated to kill cells that present the infection- or cancer-derived peptide. In this way, the immune system can mount a targeted response to infection or cancer.

Because the sequence of a TCR is important to its ability to recognize its cellular target, it is medically and scientifically useful to have an efficient method to identify the sequence of TCRs presented by T-cells that have ability to recognize and kill cells that present peptides derived from infected or cancerous cells. However, to date, there are no high-throughput assays to identify individual T-cells with desired recognition and killing activity; thus it is difficult to identify TCRs that mediate recognition of target cells.

Antibody-secreting cells (ASCs) such as B-cells and plasma cells are also clonal, in that a single cell encodes and secretes a single species of antibody. The protein sequence of the antibody, defined by the corresponding cellular DNA, determines the binding specificity of the antibody. To date, there are no high-throughput assays to identify individual cells that secrete antibody with desired target specificity and functional activity. Thus, it is difficult to identify the DNA sequence that encodes these antibodies of interest.

US 2010/092955 A1 discloses methods of screening immune cells using gel microdrops and optically distinguishable vital dyes.
US 2004/241759 A1 concerns high throughput screening of libraries.

### SUMMARY

The current invention is defined, *inter alia,* by the following items:
1. A method of determining a sequence of an immune cell receptor, the method comprising: encapsulating immune cells and target cells in microfluidic droplets contained within a microfluidic channel such that at least some of the microfluidic droplets contain at least one immune cell and at least one target cell; determining viability of the target cell after exposure of the target cell to the immune cell; separating the microfluidic droplets on the basis of the viability of the target cell by determining a signaling entity within the droplets, wherein the signaling entity is leaked from the target cell containing therein said signaling entity; and for the microfluidic droplets containing at least one immune cell and at least one non-viable target cell, determining a receptor sequence of the at least one immune cell.
2. The method of item 1, wherein the immune cells comprise:
   (a) T-cells, preferably CD8+ T-cells; and/or
   (b) B-cells; and/or
   (c) cancer cells; and/or
   (d) virally-infected cells.
3. The method of item 1 or item 2, wherein the immune cells and the target cells arise from the same organism or different organisms.
4. The method of any one of items 1-3, wherein the immune cells are human.
5. The method of any one of items 1-4, wherein the microfluidic droplets have a distribution in diameters such that no more than 5% of the droplets have a diameter greater than about 110% and/or less than about 90% of the overall average cross-sectional dimension of the droplets.
6. The method of any one of items 1-5, wherein the microfluidic channel has an average cross-sectional dimension of less than about 1 mm.
7. The method of any one of items 1-6, wherein at least about 80% of the microfluidic droplets contain at least one immune cell and at least one target cell, preferably wherein at least about 95% of the microfluidic droplets contain at least one immune cell and at least one target cell.
8. The method of any one of items 1-7, wherein the signaling entity is fluorescent and/or wherein the signaling entity is calcein and/or a calcein derivative.
9. The method of item 8, further comprising inserting the signaling entity into at least some of the target cells.
10. The method of any one of items 1-9, wherein in at least some of the microfluidic droplets, the immune cell directly kills the target cell by phagocytosis.
11. The method of any one of items 1-10, wherein in at least some of the microfluidic droplets, the immune cell kills the target cell by secreting a substance that kills the target cell, preferably wherein the substance is a cytolytic protein, preferably wherein the cytolitc protein is perforin, and/or preferably wherein the substance comprises a granzyme.
12. The method of any one of items 1-11, wherein determining a receptor sequence comprises sequencing at least a portion of the DNA within the immune cells, preferably using PCR.
13. The method of any one of items 1-12, comprising encapsulating immune cells and target cells at a rate of at least 1 droplet/s.
14. The method of any one of items 1-13, further comprising culturing cells from at least some of the microfluidic droplets containing at least one immune cell and at least one non-viable target cell.

The present invention generally relates to fluidic droplets, and to techniques for screening or sorting such fluidic droplets. In some embodiments, the fluidic droplets may contain cells such as immune cells, which can be analyzed to determine receptor sequences or other useful properties of the cells. The subject matter of the present description involves, in some cases, interrelated products, alternative solutions to a particular problem, and/or a plurality of different uses of one or more systems and/or articles.

In one aspect, the present description is generally directed to a method of determining immune cell receptors. According to a first set of embodiments, the method comprises encapsulating immune cells and target cells in microfluidic droplets contained within a microfluidic channel such that at least some of the microfluidic droplets contain at least one immune cell and at least one target cell; determining viability of the target cell after exposure of the target cell to the immune cell; separating the microfluidic droplets on the basis of the viability of the target cell; and for the microfluidic droplets containing at least one immune cell and at least one non-viable target cell, determining a receptor sequence of the at least one immune cell.

The method, in another set of embodiments, includes determining viability of target cells contained within a plurality of microfluidic droplets, at least some of which contain at least one immune cell and at least one target cell; separating the microfluidic droplets on the basis of the viability of the target cell; and for the microfluidic droplets containing at least one immune cell and at least one non-viable target cell, determining a receptor sequence of the at least one immune cell.

In yet another set of embodiments, the method includes acts of determining viability of target cells contained within a plurality of microfluidic droplets, at least some of which contain at least one effector cell and at least one target cell, wherein the effector cell interacts with the target cell to produce determinable change in the target cell; separating the microfluidic droplets on the basis of the viability of the target cell; and for the microfluidic droplets containing at least one effector cell and at least one non-viable target cell, determining a receptor sequence of the at least one effector cell.

According to still another set of embodiments, the method includes acts of encapsulating effector cells and target cells in microfluidic droplets contained within a microfluidic channel such that at least some of the microfluidic droplets contain at least one effector cell and at least one target cell, wherein the effector cell interacts with the target cell to produce determinable change in the target cell; determining viability of the target cell after exposure of the target cell to the effector cell; separating the microfluidic droplets on the basis of the viability of the target cell; and for the microfluidic droplets containing at least one effector cell and at least one non-viable target cell, determining a receptor sequence of the at least one effector cell.

In another aspect, the present description is generally directed to a method of determining cell proteins. The method, in one set of embodiments, comprises acts of determining viability of target cells contained within a plurality of microfluidic droplets, at least some of which contain at least one effector cell and at least one target cell, wherein the effector cell interacts with the target cell to produce determinable change in the target cell; separating the microfluidic droplets on the basis of the viability of the target cell; and for the microfluidic droplets containing at least one effector cell and at least one non-viable target cell, determining at least one protein-encoding gene sequence from the at least one effector cell.

According to another set of embodiments, the method includes acts of encapsulating effector cells and target cells in microfluidic droplets contained within a microfluidic channel such that at least some of the microfluidic droplets contain at least one effector cell and at least one target cell, wherein the effector cell interacts with the target cell to produce determinable change in the target cell; determining viability of the target cell after exposure of the target cell to the effector cell; separating the microfluidic droplets on the basis of the viability of the target cell; and for the microfluidic droplets containing at least one effector cell and at least one non-viable target cell, determining at least one protein-encoding gene sequence from the at least one effector cell.

In yet another aspect, the present description is generally directed to method comprising encapsulating immune cells and target cells in microfluidic droplets contained within a microfluidic channel such that at least some of the microfluidic droplets contain at least one immune cell and at least one target cell; determining viability of the target cell after exposure of the target cell to the immune cell; separating the microfluidic droplets on the basis of the viability of the target cell; and culturing cells from at least some of the microfluidic droplets containing at least one immune cell and at least one non-viable target cell.

The method, in still another aspect, includes acts of determining viability of target cells contained within a plurality of microfluidic droplets, at least some of which contain at least one immune cell and at least one target cell; separating the microfluidic droplets on the basis of the viability of the target cell; and culturing cells from at least some of the microfluidic droplets containing at least one immune cell and at least one non-viable target cell.

In yet another aspect, the droplets may contain an antibody-secreting cell, virus particles, and a target cell that can be infected by the virus. Intra-droplet assays can be used to identify cells that secrete antibody which neutralizes the virus, i.e., prevents or reduce viral infection of the target cell.

In still another aspect, the present description is generally directed to a method comprising encapsulating effector cells and target cells in microfluidic droplets contained within a microfluidic channel such that at least some of the microfluidic droplets contain at least one effector cell and at least one target cell, wherein the effector cell interacts with the target cell to produce a determinable change in the target cell, determining secretion of a substance from the effector cell after exposure of the target cell to the effector cell, separating the microfluidic droplets on the basis of the substance, and for the microfluidic droplets containing at least one effector cell and at least one non-viable target cell, determining a receptor sequence of the at least one effector cell.

In certain cases, the droplets may be used to isolate cells that secrete anti-microbial compounds, peptides, or proteins. In some embodiments, the droplets may be analyzed, e.g., using intra-droplet assays, to determine substances secreted by the cells. As an example, droplets might contain individual yeast cells that have the potential to synthesize anti-microbial agents (for example, penicillin), and microbial cells that are known human pathogens. Intra-droplet assays can be used to identify yeast cells that secrete compounds, peptides, or proteins that kill or inhibit growth of, the pathogenic microbe. In a further embodiment, prior to encapsulation, the yeast might be genetically programmed to secrete a library of peptides or proteins, and the intra-droplet screening methods would be employed to isolate the yeast that secrete an antimicrobial agent. Standard molecular biology methods can then be used to identify the DNA sequence that encodes the antimicrobial compound.

In a related example, the intra-drop assays might be used to identify cells that secrete antimicrobial compounds that are not directly encoded by DNA, i.e., non-protein or non-RNA compounds. This would allow access to chemical reagents synthesized by yeast or other organisms. For example, a yeast with anti-microbial properties would be isolated, grown to generate many clonal copies of the isolated cell, and then the cells would be broken and contents analyzed. By comparing the contents of yeast that have anti-microbial properties with the contents of yeast that do not, the anti-microbial compounds, and/or the corresponding synthesis pathways, might be identified.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
Fig. 1 is a schematic diagram illustrating a method of determining T-cell receptors, in one embodiment of the invention;
Fig. 2 illustrates droplets containing target and effector cells, in accordance with another embodiment of the invention;
Fig. 3 is a photomicrograph of an effector cell and a target cell contained in a droplet, in still another embodiment of the invention;
Figs. 4A-4D are a time sequence of photomicrographs illustrating the killing of a target cell by an effector cell, in yet another embodiment of the invention;
Figs. 5A-5C are schematic diagrams illustrating methods of sorting, in another embodiment of the invention; and
Fig. 6 illustrates a reduction in infection of cells in a droplet, in still another embodiment of the invention.

### DETAILED DESCRIPTION

The present invention generally relates to fluidic droplets, and to techniques for screening or sorting such fluidic droplets. In some embodiments, the fluidic droplets may contain cells such as immune cells, which can be analyzed to determine receptor sequences or other useful properties of the cells. For example, in one aspect, the present invention is generally related to determining immune cell receptors by encapsulating immune cells and target cells in microfluidic droplets, determining the effect of the immune cells on the target cells, and for those immune cells that kill or otherwise adversely affect the target cells, determining one or more receptor sequences of those immune cells. The target cells may be, for example, cancer cells or virally-infected cells. In some cases, the receptor sequences can be used, for example, to identify certain properties of the immune cells, to screen for drugs or other therapeutic agents, or the like.

Certain aspects of the description are generally directed to assays where two (or more) cells are contained within a droplet, such as a microfluidic droplet. One of the cells may act on other cells within the droplet. Droplets where a certain result occurs (e.g., a cell is killed by another cell) may be separated from droplets where other results (or no result) occur. The cells contained within the separated droplets may then be determined or analyzed, for example, by sequencing the cells' DNA, analyzing cellular mRNA levels, analyzing cell protein modifications, e.g., protein phosphorylation levels, etc.

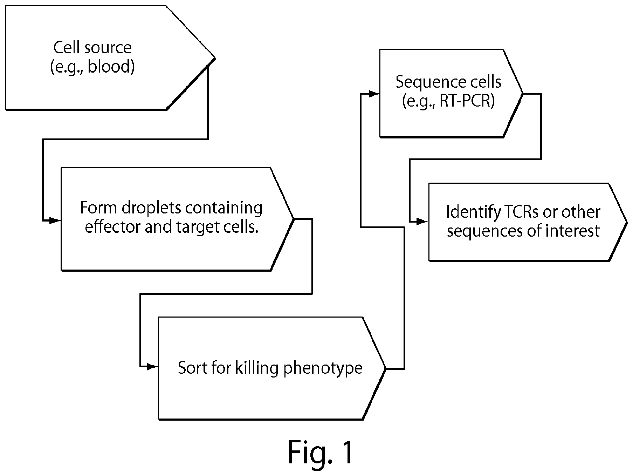

For example, referring to Fig. 1, in one set of embodiments, two (or more) cells are contained within a droplet. The cells may arise from the same organism, different organisms of the same species, different organisms of different species (e.g., human and mouse cells, human and rat cells, human cells and bacteria, human cells and fungal cells, rat cells and fungal cells, bacteria and fungal cells, etc.), or the like. The cells may also be genetically modified, for example, a set of cells may be transformed with a library of DNA plasmids that encode potentially functional peptides or proteins. If more than two cells are present in the droplet, some of the cells may be substantially the same (e.g., duplicate cells), and/or there may be three, four, or more types of cells contained within the droplet, and such cells may independently arise from the same species or different species, and from the same organism or different organisms. The cells may be present in a liquid which is then formed into droplets containing the cells, and/or cells may be inserted or injected into the droplets after formation of the droplets. Examples of techniques for containing cells in droplets include those disclosed in Int. Pat. Apl. No. PCT/US2004/027912, filed August 27, 2004, entitled "Electronic Control of Fluidic Species," by Link, *et al.,* published as WO 2005/021151 on March 10, 2005, or Int. Pat. Apl. No. PCT/US2010/040006, filed June 25, 2010, entitled "Fluid Injection," by Weitz, *et al.,* published as WO 2010/151776 on December 29, 2010. Other techniques known to those of ordinary skill in the art for producing a cell contained within a droplet may also be used.

In some cases, one (or more) of the cells may be an effector cell that interacts with other cells (e.g., a target cell) to produce a change in the target cell (and/or in some cases, the effector cell). For example, the effector cell may be a T-cell or other immune cell that recognizes a target cell, e.g., the T-cell may kill or inhibit the target cell. As another example, the effector cell may be a cell that secretes a substance (e.g., a growth factor, a neurotransmitter, a hormone, a signaling peptide or other signaling compound, an apoptosis inducing factor, etc.), that influences the target cell in a determinable way (e.g., by inhibiting or promoting cell growth, division, apoptosis, differentiation, etc.). In some cases, the target cell and/or the effector cell may be genetically modified, e.g., to produce or increase production of the secreted substance, although in other cases, the cells are not genetically modified.

As an example, the effector cells may interact with the target cells by producing or secreting antibodies or cytolytic proteins, or other compounds (e.g., perforins, granzymes, etc.) that are able to interact with the target cell. Other compounds that could be secreted include other gene-encoded proteins or other compounds that are not proteins, e.g., penicillins, hormones, small molecules (e.g. less than 2 kDa or 1 kDa), etc. As another example, the effector cells may kill the target cells, e.g., through phagocytosis, inducement of apoptosis, etc. The effector cells may also release signaling molecules or other substances that interact with the target cell, as mentioned above. Examples of effector cells include, but are not limited to, immune cells such as B-cells, T-cells, natural killer cells, lymphocytes, macrophages, neutrophils, basophils, mast cells, or the like. In one set of embodiments, for instance, the effector cell is a CD8+ T-cell. Non-limiting examples of target cells include cancer cells (or cells suspected of being cancerous), normal cells, foreign cells (e.g., bacteria, fungi, pathogens, etc.), viruses, or the like. In some cases, the target cell may be an infected cell, e.g., a cell infected with a bacterium, a virus, a fungus, a pathogen, or the like. As mentioned, the interaction between the effector cell and the target cell may be direct (e.g., the effector cell binds directly to the target cell), and/or indirect (e.g., the target cell may secrete a substance that affects the target cell). As non-limiting examples, T-cells and tumor cells from a cancer patient could be studied to determine those receptors on the T-cells that are able to recognize such tumor cells, or a T-cell may be associated with cells infected with a pathogen (e.g., a bacterium or a virus) to determine those receptors able to recognize the pathogen.

The cells may arise from any suitable species, and as mentioned, cells from more than one species may be present within a droplet. For example, effector cells and target cells may come from the same species or different species. The cells may include a eukaryotic cell, an animal cell, a plant cell, a bacterium or other single-cell organism, etc. If the cell is an animal cell, the cell may be, for example, an invertebrate cell (e.g., a cell from a fruit fly), a fish cell (e.g., a zebrafish cell), an amphibian cell (e.g., a frog cell), a reptile cell, a bird cell, or a human or non-human mammal, such as a monkey, ape, cow, sheep, goat, horse, rabbit, pig, mouse, rat, guinea pig, dog, cat, etc. If the cell is from a multicellular organism, the cell may be from any part of the organism. For instance, if the cell is from an animal, the cell may be an immune cell, a cardiac cell, a fibroblast, a keratinocyte, a heptaocyte, a chondracyte, a neural cell, an osteocyte, an osteoblast, a muscle cell, a blood cell, an endothelial cell, or the like. In some cases, the cell is genetically engineered.

In some cases, the target cell may contain or be exposed to a signaling entity. The signaling entity may be any entity that can be determined in some fashion using a detection method. For example, the signaling entity may be a fluorescent entity that is released from the target cell upon death or rupture of the target cell, or the signaling entity may be an inhibitor of a fluorescent signal. Thus, the droplets can be determined to determine the signaling entity (e.g., by determining fluorescence). For example, the distribution and/or intensity of the signaling entity may be determined within the droplet, and the droplet sorted on the basis of the signaling entity. Examples of sorting techniques are discussed in more detail below. For example, viability of target cells may be determined by determining leakage of signaling entity from the target cells, and the cells sorted based on such determinations. As another example, a state or characteristic of the target cell (e.g., its internal pH or its size) may be determined using the signaling entity. As another example, the target cell may respond to a signaling entity by synthesizing a measurable species, such as a particular mRNA or protein. In some cases, the measurable synthesized protein may be a marker protein such as Green Fluorescent Protein (GFP).

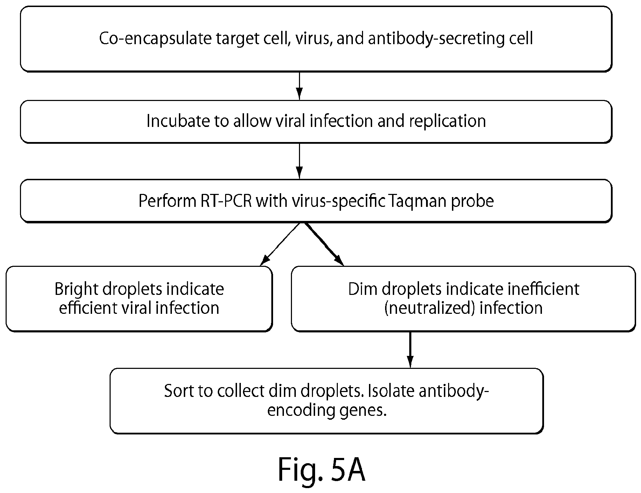

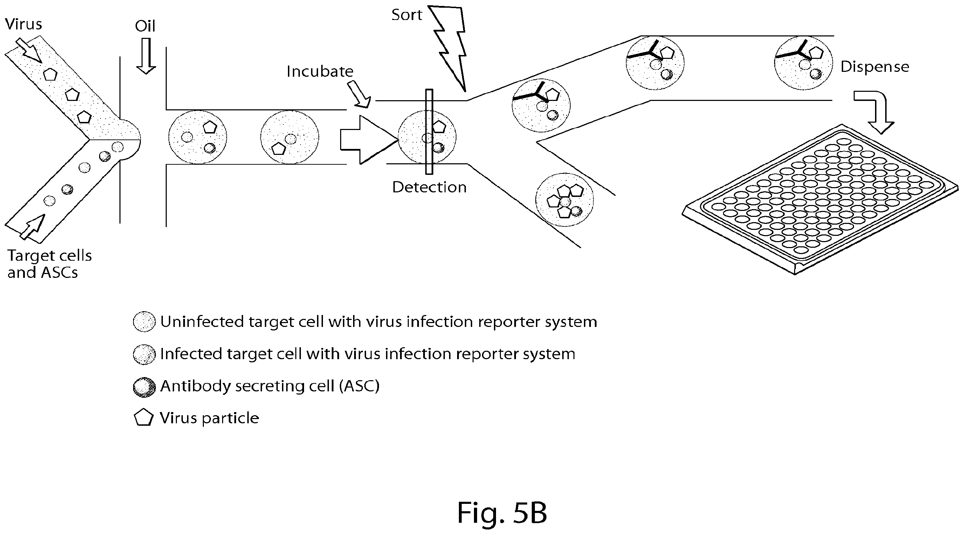

As an example, in some cases, antibody secreted by an encapsulated antibody-secreting cell (ASC) may inhibit viral infection of a co-encapsulated target cell. Non-limiting examples of antibody-secreting cells include hybridoma cells, B-cells, plasma cells, or the like. Inhibition of viral infection may be determined by survival of the target cell. In some cases, target cells can be genetically modified to provide a fluorescent indicator of viral infection. In some embodiments, droplets in which target cells remain non-fluorescent are likely to contain an ASC that secretes an antibody that inhibits viral infection. Droplets in which viral infection is inhibited may be selected by microfluidic sorting so that the sequence of the neutralizing antibodies can be determined. For example, referring to Fig. 5A, a target cell, a virus, and an antibody-secreting cell may be contained within a droplet. Droplets where the antibody-secreting cells are able to at least partially neutralize the virus may be separated from those droplets where the antibody-secreting cells did not neutralize the virus. In some embodiments, controls containing irrelevant cells (e.g., irrelevant antibody-secreting cells) may also be used to show neutralization of the viruses is due to the antibodies produced by the antibody-secreting cells. In Fig. 5B, a microfluidic system for sorting is schematically depicted.

The signaling entity may be fluorescent in some cases. As other non-limiting examples, the signaling entity may be a dye, a radioactive atom or compound, etc. The signaling entity may also be an ultraviolet dye or an infrared dye in some cases. Examples of signaling entities used to determine the status or condition of the target cell include, but are not limited to, calcein (or calcein derivatives such calcein AM), propidium iodide, 7-aminoactinomycin D, nuclear stains, Calcein Blue AM, Calcein Violet AM, Fura-2 AM, Indo-1 AM, resazurin, and the like. Many such dyes are commercially available. In some embodiments, the signaling entity may be inserted into the target cells using any suitable technique known to those of ordinary skill in the art, e.g., transfection, electroporation, etc. Thus, determination of the signaling entity may occur using techniques such as radioactivity, fluorescence, phosphorescence, light scattering, light absorption, fluorescence polarization, or the like. Many detectors that operate using such principles are commercially available. The detector may be used to determine at least one of the signaling entities that may be present, and in some cases, more than one detector may be used.

In some cases, at least about 30%, at least about 40%, at least about 50%, at least about 60%, at least about 70%, at least about 75%, at least about 80%, at least about 85%, at least about 90%, or at least about 95% of the effector cells that are studied within a droplet (or within a population of droplets) are the same type of cell. In some cases, at least about 30%, at least about 40%, at least about 50%, at least about 60%, at least about 70%, at least about 75%, at least about 80%, at least about 85%, at least about 90%, or at least about 95% of the target cells within a droplet (or within a population of droplets) are the same type of cell. However, in other embodiments, populations of target and/or effector cells may be studied. A single type of effector cell may be studied against a range of different types of target cells, or a single target cell may be tested against a range of different types of effector cells, for instance. As non-limiting examples, the target cells may arise from one or more organs from a single organism, and/or from different organisms of the same or different species. In other examples, the target cells may arise from a population of different bacteria or other organisms, e.g., from an infection, a tumor, a soil sample, a water sample, etc.

In one set of embodiments, as a non-limiting example, a droplet can be formed containing a T-cell as an effector cell, and a cancer cell as a target cell. In some cases, the cancer cell may contain therein a signaling entity, such as calcein (which is fluorescent). The T-cell may be allowed to interact with the cancer cell, and in some cases, the T-cell may have suitable receptors that allow it to recognize the cancer cell as a cancer cell. The T-cell may then proceed to kill the cancer cell, thereby causing release of calcein from the cancer cell into the droplet. The droplet may then be analyzed to determine its fluorescence; for example, no detected fluorescence or relatively highly localized fluorescence within a droplet may indicate that the cancer cell is still alive or intact, while more diffused fluorescence within the droplet may indicate that the cancer cell has been killed (presumably by the T-cell). Accordingly, by sorting the droplets on the basis of their fluorescence, T-cells that recognized the cancer cells may be separated from T-cells that did not recognized the cancer cells (e.g., due to having incorrect receptors, being too weak to attack the cancer cells, cancer cells having characteristics that allow them to evade the T-cells, etc.). The T-cells that were successful at killing cancer cells may then be analyzed using any suitable technique. For example, the T-cells may be sequenced using techniques known to those of ordinary skill in the art to identify the receptors that allowed the T-cells to recognize and attack the cancer cells. As a non-limiting example, various receptors for cell recognition may be determined or sequenced using such techniques.

In some cases, relatively large numbers of droplets may be created that contain the same type and/or numbers of cells therein. For example, a population of at least 10, at least 30, at least 50, at least 100, at least 300, at least 500, at least 1,000, at least 3,000, at least 5,000, at least 10,000, at least 30,000, at least 50,000, at least 100,000 droplets, at least 300,000 droplets, at least 500,000 droplets, at least 1,000,000 droplets, at least 3,000,000 droplets, at least 5,000,000 droplets, at least 10,000,000 droplets, etc., containing cells (e.g., one or more effector cells and/or one or more target cells) may be created. In some cases, at least about 30%, at least about 40%, at least about 50%, at least about 60%, at least about 70%, at least about 75%, at least about 80%, at least about 85%, at least about 90%, or at least about 95% of the droplets that are created may contain the same number of cells (e.g., 2 cells), and/or the same number of types of cells (e.g., a particular effector cell and a particular target cell). For example, the droplets may each contain at least one immune cell and at least one target cell (e.g., a cancer cell or an infected cell) for the immune cell.

In one set of embodiments, cells sorted for a desired characteristic (e.g., ability to kill a target cell) may be analyzed to determine various characteristics of the cells. For example, receptors on the cells may be determined or sequenced, e.g., to determine receptors able to interact with the target cells. As other examples, sequences encoding antibodies able to interact with the target cells, and/or sequences encoding substances that are secreted to interact with the target cells, may be analyzed. In some cases, all, or a portion of, the DNA or the genome of the effector cells may be sequenced or otherwise determined. The cells may also be grown or expanded (e.g., in number) before such determination, in some embodiments, e.g., using cell culture techniques known to those of ordinary skill in the art. In some cases, the cells that are sorted may be purified and cultured, e.g., for applications as discussed herein, for subsequent study, or for other applications or uses.

The DNA from the cell may be sequenced using any suitable technique known to those of ordinary skill in the art. Examples of DNA sequencing techniques include, but are not limited to, PCR (polymerase chain reaction), "sequencing by synthesis" techniques (e.g., using DNA synthesis by DNA polymerase to identify the bases present in the complementary DNA molecule), "sequencing by ligation" (e.g., using DNA ligases), "sequencing by hybridization" (e.g., using DNA microarrays), nanopore sequencing techniques, or the like. Optionally, the extracted nucleic acid sequence may be amplified, duplicated, or expanded by PCR, rolling circle replication, or other techniques known to those of ordinary skill in the art.

As mentioned, various aspects of the description relates to systems and methods for producing droplets of fluid surrounded by a liquid. Any technique may be used to make a droplet, including those described herein. The fluid and the liquid may be essentially immiscible in many cases, i.e., immiscible on a time scale of interest (e.g., the time it takes a fluidic droplet to be transported through a particular system or device). In certain cases, the droplets may each be substantially the same shape or size, as described herein. The fluid may also contain other species, for example, certain molecular species (e.g., as discussed herein), cells, particles, etc.

In one set of embodiments, for example, electric charge may be created on a fluid surrounded by a liquid, which may cause the fluid to separate into individual droplets within the liquid. In some embodiments, the fluid and the liquid may be present in a channel, e.g., a microfluidic channel, or other constricted space that facilitates application of an electric field to the fluid (which may be "AC" or alternating current, "DC" or direct current etc.), for example, by limiting movement of the fluid with respect to the liquid. Thus, the fluid can be present as a series of individual charged and/or electrically inducible droplets within the liquid. In one embodiment, the electric force exerted on the fluidic droplet may be large enough to cause the droplet to move within the liquid. In some cases, the electric force exerted on the fluidic droplet may be used to direct a desired motion of the droplet within the liquid, for example, to or within a channel or a microfluidic channel (e.g., as further described herein), etc.

Electric charge may be created in the fluid within the liquid using any suitable technique, for example, by placing the fluid within an electric field (which may be AC, DC, etc.), and/or causing a reaction to occur that causes the fluid to have an electric charge, for example, a chemical reaction, an ionic reaction, a photocatalyzed reaction, etc. In one embodiment, the fluid is an electrical conductor. As used herein, a "conductor" is a material having a conductivity of at least about the conductivity of 18 megohm (MOhm or MΩ) water. The liquid surrounding the fluid may have a conductivity less than that of the fluid. For instance, the liquid may be an insulator, relative to the fluid, or at least a "leaky insulator," i.e., the liquid is able to at least partially electrically insulate the fluid for at least a short period of time. Those of ordinary skill in the art will be able to identify the conductivity of fluids. In one non-limiting embodiment, the fluid may be substantially hydrophilic, and the liquid surrounding the fluid may be substantially hydrophobic.

The electric field, in some embodiments, is generated from an electric field generator, i.e., a device or system able to create an electric field that can be applied to the fluid. The electric field generator may produce an AC field (i.e., one that varies periodically with respect to time, for example, sinusoidally, sawtooth, square, etc.), a DC field (i.e., one that is constant with respect to time), a pulsed field, etc. The electric field generator may be constructed and arranged to create an electric field within a fluid contained within a channel or a microfluidic channel. The electric field generator may be integral to or separate from the fluidic system containing the channel or microfluidic channel, according to some embodiments. As used herein, "integral" means that portions of the components integral to each other are joined in such a way that the components cannot be manually separated from each other without cutting or breaking at least one of the components.

In another set of embodiments, droplets of fluid can be created from a fluid surrounded by a liquid within a channel by altering the channel dimensions in a manner that is able to induce the fluid to form individual droplets. The channel may, for example, be a channel that expands relative to the direction of flow, e.g., such that the fluid does not adhere to the channel walls and forms individual droplets instead, or a channel that narrows relative to the direction of flow, e.g., such that the fluid is forced to coalesce into individual droplets. In other embodiments, internal obstructions may also be used to cause droplet formation to occur. For instance, baffles, ridges, posts, or the like may be used to disrupt liquid flow in a manner that causes the fluid to coalesce into fluidic droplets.

In some cases, the channel dimensions may be altered with respect to time (for example, mechanically or electromechanically, pneumatically, etc.) in such a manner as to cause the formation of individual fluidic droplets to occur. For example, the channel may be mechanically contracted ("squeezed") to cause droplet formation, or a fluid stream may be mechanically disrupted to cause droplet formation, for example, through the use of moving baffles, rotating blades, or the like. Other examples of methods for creating droplets include those disclosed in Int. Pat. Apl. No. PCT/US2003/020542, filed June 30, 2003, entitled "Method and Apparatus for Fluid Dispersion," by Stone, *et al.,* published as WO 2004/002627 on January 8, 2004.

In some instances, the droplets may be created at relatively high rates. For instance, at least about 1 droplet per second may be created in some cases, and in other cases, at least about 10 droplets per second, at least about 20 droplets per second, at least about 30 droplets per second, at least about 100 droplets per second, at least about 200 droplets per second, at least about 300 droplets per second, at least about 500 droplets per second, at least about 750 droplets per second, at least about 1000 droplets per second, at least about 1500 droplets per second, at least about 2000 droplets per second, at least about 3000 droplets per second, at least about 5000 droplets per second, at least about 7500 droplets per second, at least about 10,000 droplets per second, at least about 15,000 droplets per second, at least about 20,000 droplets per second, at least about 30,000 droplets per second, at least about 50,000 droplets per second, at least about 75,000 droplets per second, at least about 100,000 droplets per second, at least about 150,000 droplets per second, at least about 200,000 droplets per second, at least about 300,000 droplets per second, at least about 500,000 droplets per second, at least about 750,000 droplets per second, at least about 1,000,000 droplets per second, at least about 1,500,000 droplets per second, at least about 2,000,000 or more droplets per second, or at least about 3,000,000 or more droplets per second may be created.

Other examples of the production of droplets of fluid surrounded by a liquid are described in International Patent Application Serial No. PCT/US2004/010903, filed April 9, 2004 by Link, et al., published as WO 2004/091763 on October 28, 2004, and International Patent Application Serial No. PCT/US03/20542, filed June 30, 2003 by Stone, et al.*,* published as WO 2004/002627 on January 8, 2004.

In some embodiments, a species (for example, a cell) may be contained within the droplet, e.g., before or after formation. In some cases, more than one species may be present. Thus, for example, a precise quantity of a drug, pharmaceutical, or other agent can be contained within a droplet, e.g., in addition to a cell. For example, the species may be drug or other species that is suspected of being able to affect the interaction between an effector cell and a target cell within a droplet. Other species that can be contained within a droplet include, for example, biochemical species such as nucleic acids such as siRNA, mRNA, RNAi and DNA, proteins, peptides, or enzymes, or the like. Additional species that can be contained within a droplet include, but are not limited to, nanoparticles, quantum dots, proteins, indicators, dyes, fluorescent species, chemicals, amphiphilic compounds, detergents, drugs, or the like. Further examples of species that can be contained within a droplet include, but are not limited to, growth regulators, vitamins, hormones, or microbicides.

In certain instances, the description provides for the production of droplets consisting essentially of a substantially uniform number of entities of a species therein (i.e., molecules, cells, particles, etc.). For example, about 90%, about 93%, about 95%, about 97%, about 98%, or about 99%, or more of a plurality or series of droplets may each contain the same number of entities of a particular species. For instance, a substantial number of fluidic droplets produced, e.g., as described above, may each contain 1 entity, 2 entities, 3 entities, 4 entities, 5 entities, 7 entities, 10 entities, 15 entities, 20 entities, 25 entities, 30 entities, 40 entities, 50 entities, 60 entities, 70 entities, 80 entities, 90 entities, 100 entities, etc., where the entities are molecules or macromolecules, cells, particles, etc. In some cases, the droplets may each independently contain a range of entities, for example, less than 20 entities, less than 15 entities, less than 10 entities, less than 7 entities, less than 5 entities, or less than 3 entities in some cases.

As discussed, in some aspects, fluidic droplets may be screened and/or sorted, and in some cases, at relatively high rates. For example, a characteristic of a droplet may be sensed and/or determined in some fashion (e.g., as herein described), then the droplet may be directed towards a particular region of the device, for example, for sorting or screening purposes. For example, the fluidic droplets may be sorted into two or more than two channels, e.g., based on interaction of the cells within the droplets. In some embodiments, a characteristic of a fluidic droplet may be sensed and/or determined in some fashion, for example, as described herein (e.g., fluorescence of the fluidic droplet may be determined), and, in response, an electric field may be applied or removed from the fluidic droplet to direct the fluidic droplet to a particular region (e.g. a channel). Other techniques for sensing cells and/or for sorting cells that are known to those of ordinary skill in the art may also be used, in some embodiments of the invention.

In some cases, high sorting speeds may be achievable using certain systems and methods of the invention. For instance, at least about 1 droplet per second may be determined and/or sorted in some cases, and in other cases, at least about 10 droplets per second, at least about 20 droplets per second, at least about 30 droplets per second, at least about 100 droplets per second, at least about 200 droplets per second, at least about 300 droplets per second, at least about 500 droplets per second, at least about 750 droplets per second, at least about 1000 droplets per second, at least about 1500 droplets per second, at least about 2000 droplets per second, at least about 3000 droplets per second, at least about 5000 droplets per second, at least about 7500 droplets per second, at least about 10,000 droplets per second, at least about 15,000 droplets per second, at least about 20,000 droplets per second, at least about 30,000 droplets per second, at least about 50,000 droplets per second, at least about 75,000 droplets per second, at least about 100,000 droplets per second, at least about 150,000 droplets per second, at least about 200,000 droplets per second, at least about 300,000 droplets per second, at least about 500,000 droplets per second, at least about 750,000 droplets per second, at least about 1,000,000 droplets per second, at least about 1,500,000 droplets per second, at least about 2,000,000 or more droplets per second, or at least about 3,000,000 or more droplets per second may be determined and/or sorted in such a fashion.

In one set of embodiments, a fluidic droplet may be directed by creating an electric charge (e.g., as previously described) on the droplet, and steering the droplet using an applied electric field, which may be an AC field, a DC field, etc. As an example, an electric field may be selectively applied and removed (or a different electric field may be applied, e.g., a reversed electric field) as needed to direct the fluidic droplet to a particular region. The electric field may be selectively applied and removed as needed, in some embodiments, without substantially altering the flow of the liquid containing the fluidic droplet. For example, a liquid may flow on a substantially steady-state basis (i.e., the average flowrate of the liquid containing the fluidic droplet deviates by less than 20% or less than 15% of the steady-state flow or the expected value of the flow of liquid with respect to time, and in some cases, the average flowrate may deviate less than 10% or less than 5%) or other predetermined basis through a fluidic system of the invention (e.g., through a channel or a microchannel), and fluidic droplets contained within the liquid may be directed to various regions, e.g., using an electric field, without substantially altering the flow of the liquid through the fluidic system.

In another set of embodiments, a fluidic droplet may be sorted or steered by inducing a dipole in the fluidic droplet (which may be initially charged or uncharged), and sorting or steering the droplet using an applied electric field. The electric field may be an AC field, a DC field, etc.

In other embodiments, however, the fluidic droplets may be screened or sorted within a fluidic system of the disclosure by altering the flow of the liquid containing the droplets. For instance, in one set of embodiments, a fluidic droplet may be steered or sorted by directing the liquid surrounding the fluidic droplet into a first channel, a second channel, etc.

In another set of embodiments, pressure within a fluidic system, for example, within different channels or within different portions of a channel, can be controlled to direct the flow of fluidic droplets. For example, a droplet can be directed toward a channel junction including multiple options for further direction of flow (e.g., directed toward a branch, or fork, in a channel defining optional downstream flow channels). Pressure within one or more of the optional downstream flow channels can be controlled to direct the droplet selectively into one of the channels, and changes in pressure can be effected on the order of the time required for successive droplets to reach the junction, such that the downstream flow path of each successive droplet can be independently controlled. In one arrangement, the expansion and/or contraction of liquid reservoirs may be used to steer or sort a fluidic droplet into a channel, e.g., by causing directed movement of the liquid containing the fluidic droplet. The liquid reservoirs may be positioned such that, when activated, the movement of liquid caused by the activated reservoirs causes the liquid to flow in a preferred direction, carrying the fluidic droplet in that preferred direction. For instance, the expansion of a liquid reservoir may cause a flow of liquid towards the reservoir, while the contraction of a liquid reservoir may cause a flow of liquid away from the reservoir. In some cases, the expansion and/or contraction of the liquid reservoir may be combined with other flow-controlling devices and methods, e.g., as described herein. Non-limiting examples of devices able to cause the expansion and/or contraction of a liquid reservoir include pistons and piezoelectric components. In some cases, piezoelectric components may be particularly useful due to their relatively rapid response times, e.g., in response to an electrical signal.

In certain aspects of the description, sensors are provided that can sense and/or determine one or more characteristics of the fluidic droplets, and/or a characteristic of a portion of the fluidic system containing the fluidic droplet (e.g., the liquid surrounding the fluidic droplet) in such a manner as to allow the determination of one or more characteristics of the fluidic droplets. Characteristics determinable with respect to the droplet and usable in the invention can be identified by those of ordinary skill in the art. Non-limiting examples of such characteristics include fluorescence, spectroscopy (e.g., optical, infrared, ultraviolet, etc.), radioactivity, mass, volume, density, temperature, viscosity, pH, concentration of a substance, such as a biological substance (e.g., a protein, a nucleic acid, etc.), or the like.

In some cases, the sensor may be connected to a processor, which in turn, cause an operation to be performed on the fluidic droplet, for example, by sorting the droplet, adding or removing electric charge from the droplet, fusing the droplet with another droplet, etc. One or more sensors and/or processors may be positioned to be in sensing communication with the fluidic droplet. "Sensing communication," as used herein, means that the sensor may be positioned anywhere such that the fluidic droplet within the fluidic system (e.g., within a channel), and/or a portion of the fluidic system containing the fluidic droplet may be sensed and/or determined in some fashion. For example, the sensor may be in sensing communication with the fluidic droplet and/or the portion of the fluidic system containing the fluidic droplet fluidly, optically or visually, thermally, pneumatically, electronically, or the like. The sensor can be positioned proximate the fluidic system, for example, embedded within or integrally connected to a wall of a channel, or positioned separately from the fluidic system but with physical, electrical, and/or optical communication with the fluidic system so as to be able to sense and/or determine the fluidic droplet and/or a portion of the fluidic system containing the fluidic droplet (e.g., a channel or a microchannel, a liquid containing the fluidic droplet, etc.). For example, a sensor may be free of any physical connection with a channel containing a droplet, but may be positioned so as to detect electromagnetic radiation arising from the droplet or the fluidic system, such as infrared, ultraviolet, or visible light. The electromagnetic radiation may be produced by the droplet, and/or may arise from other portions of the fluidic system (or externally of the fluidic system) and interact with the fluidic droplet and/or the portion of the fluidic system containing the fluidic droplet in such as a manner as to indicate one or more characteristics of the fluidic droplet, for example, through absorption, reflection, diffraction, refraction, fluorescence, phosphorescence, changes in polarity, phase changes, changes with respect to time, etc. As an example, a laser may be directed towards the fluidic droplet and/or the liquid surrounding the fluidic droplet, and the fluorescence of the fluidic droplet and/or the surrounding liquid may be determined. "Sensing communication," as used herein may also be direct or indirect. As an example, light from the fluidic droplet may be directed to a sensor, or directed first through a fiber optic system, a waveguide, etc., before being directed to a sensor.

Non-limiting examples of sensors useful in the invention include optical or electromagnetically-based systems. For example, the sensor may be a fluorescence sensor (e.g., stimulated by a laser), a microscopy system (which may include a camera or other recording device), or the like. As another example, the sensor may be an electronic sensor, e.g., a sensor able to determine an electric field or other electrical characteristic. For example, the sensor may detect capacitance, inductance, etc., of a fluidic droplet and/or the portion of the fluidic system containing the fluidic droplet.

As used herein, a "processor" or a "microprocessor" is any component or device able to receive a signal from one or more sensors, store the signal, and/or direct one or more responses (e.g., as described above), for example, by using a mathematical formula or an electronic or computational circuit. The signal may be any suitable signal indicative of the environmental factor determined by the sensor, for example a pneumatic signal, an electronic signal, an optical signal, a mechanical signal, etc.

As a particular non-limiting example, a device of the description may contain fluidic droplets containing one or more cells. The cells may be exposed to a signaling entity, such as a fluorescent signal marker that binds if a certain condition is present, for example, the marker may bind to a first cell type but not a second cell type, the marker may bind to an expressed protein, the marker may indicate viability of the cell (i.e., if the cell is alive or dead), the marker may be indicative of the state of development or differentiation of the cell, etc., and the cells may be directed through a fluidic system of the description based on the presence/absence, and/or magnitude of the fluorescent signal marker. For instance, determination of the fluorescent signal marker may cause the cells to be directed to one region of the device (e.g., a collection chamber), while the absence of the fluorescent signal marker may cause the cells to be directed to another region of the device (e.g., a waste chamber). Thus, in this example, a population of cells may be screened and/or sorted on the basis of one or more determinable or targetable characteristics of the cells, for example, to select live cells, cells expressing a certain protein, a certain cell type, etc.

As mentioned, certain aspects of the description are directed to the production of droplets using apparatuses and devices such as those described herein, for example, within microfluidic channels or other microfluidic systems. In some cases, e.g., with relatively large numbers of side channels, relatively large droplet production rates may be achieved. For instance, in some cases, greater than about 1,000 droplets/s, greater than or equal to 5,000 droplets/s, greater than about 10,000 droplets/s, greater than about 50,000 droplets/s, greater than about 100,000 droplets/s, greater than about 300,000 droplets/s, greater than about 500,000 droplets/s, or greater than about 1,000,000 droplets/s, etc. may be produced.

In addition, in some cases, a plurality of droplets may be produced that are substantially monodisperse, in some embodiments. In some cases, the plurality of droplets may have a distribution of characteristic dimensions such that no more than about 20%, no more than about 18%, no more than about 16%, no more than about 15%, no more than about 14%, no more than about 13%, no more than about 12%, no more than about 11%, no more than about 10%, no more than about 5%, no more than about 4%, no more than about 3%, no more than about 2%, no more than about 1%, or less, of the droplets have a characteristic dimension greater than or less than about 20%, less than about 30%, less than about 50%, less than about 75%, less than about 80%, less than about 90%, less than about 95%, less than about 99%, or more, of the average characteristic dimension of all of the droplets. Those of ordinary skill in the art will be able to determine the average characteristic dimension of a population of droplets, for example, using laser light scattering, microscopic examination, or other known techniques. In one set of embodiments, the plurality of droplets may have a distribution of characteristic dimension such that no more than about 20%, no more than about 10%, or no more than about 5% of the droplets may have a characteristic dimension greater than about 120% or less than about 80%, greater than about 115% or less than about 85%, or greater than about 110% or less than about 90% of the average of the characteristic dimension of the plurality of droplets. The "characteristic dimension" of a droplet, as used herein, is the diameter of a perfect sphere having the same volume as the droplet. In addition, in some instances, the coefficient of variation of the characteristic dimension of the exiting droplets may be less than or equal to about 20%, less than or equal to about 15%, or less than or equal to about 10%.

The average characteristic dimension or diameter of the plurality of droplets, in some embodiments, may be less than about 1 mm, less than about 500 micrometers, less than about 200 micrometers, less than about 100 micrometers, less than about 75 micrometers, less than about 50 micrometers, less than about 25 micrometers, less than about 10 micrometers, or less than about 5 micrometers in some cases. The average characteristic dimension of a droplet (or plurality of droplets) may also be greater than or equal to about 1 micrometer, greater than or equal to about 2 micrometers, greater than or equal to about 3 micrometers, greater than or equal to about 5 micrometers, greater than or equal to about 10 micrometers, greater than or equal to about 15 micrometers, or greater than or equal to about 20 micrometers in certain cases.

In some embodiments, the fluidic droplets may each be substantially the same shape and/or size. The shape and/or size can be determined, for example, by measuring the average diameter or other characteristic dimension of the droplets. The term "determining," as used herein, generally refers to the analysis or measurement of a species, for example, quantitatively or qualitatively, and/or the detection of the presence or absence of the species. "Determining" may also refer to the analysis or measurement of an interaction between two or more species, for example, quantitatively or qualitatively, or by detecting the presence or absence of the interaction.

In some embodiments, a droplet may undergo additional processes. For example, as discussed, a droplet may be sorted and/or detected. For example, a species within a droplet may be determined, and the droplet may be sorted based on that determination. In general, a droplet may undergo any suitable process known to those of ordinary skill in the art. See, e.g., Int. Pat. Apl. No. PCT/US2004/010903, filed April 9, 2004, entitled "Formation and Control of Fluidic Species," by Link, *et al.,* published as WO 2004/091763 on October 28, 2004; Int. Pat. Apl. No. PCT/US2003/020542, filed June 30, 2003, entitled "Method and Apparatus for Fluid Dispersion," by Stone, *et al.,* published as WO 2004/002627 on January 8, 2004; Int. Pat. Apl. No. PCT/US2006/007772, filed March 3, 2006, entitled "Method and Apparatus for Forming Multiple Emulsions," by Weitz, *et al.,* published as WO 2006/096571 on September 14, 2006; Int. Pat. Apl. No. PCT/US2004/027912, filed August 27, 2004, entitled "Electronic Control of Fluidic Species," by Link, *et al.,* published as WO 2005/021151 on March 10, 2005.

Certain aspects of the disclosure are generally directed to devices containing channels such as those described above. In some cases, some of the channels may be microfluidic channels, but in certain instances, not all of the channels are microfluidic. There can be any number of channels, including microfluidic channels, within the device, and the channels may be arranged in any suitable configuration. The channels may be all interconnected, or there can be more than one network of channels present. The channels may independently be straight, curved, bent, etc. In some cases, there may be a relatively large number and/or a relatively large length of channels present in the device. For example, in some embodiments, the channels within a device, when added together, can have a total length of at least about 100 micrometers, at least about 300 micrometers, at least about 500 micrometers, at least about 1 mm, at least about 3 mm, at least about 5 mm, at least about 10 mm, at least about 30 mm, at least 50 mm, at least about 100 mm, at least about 300 mm, at least about 500 mm, at least about 1 m, at least about 2 m, or at least about 3 m in some cases. As another example, a device can have at least 1 channel, at least 3 channels, at least 5 channels, at least 10 channels, at least 20 channels, at least 30 channels, at least 40 channels, at least 50 channels, at least 70 channels, at least 100 channels, etc.

In some embodiments, at least some of the channels within the device are microfluidic channels. "Microfluidic," as used herein, refers to a device, article, or system including at least one fluid channel having a cross-sectional dimension of less than about 1 mm. The "cross-sectional dimension" of the channel is measured perpendicular to the direction of net fluid flow within the channel. Thus, for example, some or all of the fluid channels in a device can have a maximum cross-sectional dimension less than about 2 mm, and in certain cases, less than about 1 mm. In one set of embodiments, all fluid channels in a device are microfluidic and/or have a largest cross sectional dimension of no more than about 2 mm or about 1 mm. In certain embodiments, the fluid channels may be formed in part by a single component (e.g. an etched substrate or molded unit). Of course, larger channels, tubes, chambers, reservoirs, etc. can be used to store fluids and/or deliver fluids to various elements or systems in other embodiments of the invention, for example, as previously discussed. In one set of embodiments, the maximum cross-sectional dimension of the channels in a device is less than 500 micrometers, less than 200 micrometers, less than 100 micrometers, less than 50 micrometers, or less than 25 micrometers, less than about 10 micrometers, less than about 5 micrometers, or less than about 1 micrometer.

A "channel," as used herein, means a feature on or in a device or substrate that at least partially directs flow of a fluid. The channel can have any cross-sectional shape (circular, oval, triangular, irregular, square or rectangular, or the like) and can be covered or uncovered. In embodiments where it is completely covered, at least one portion of the channel can have a cross-section that is completely enclosed, or the entire channel may be completely enclosed along its entire length with the exception of its inlets and/or outlets or openings. A channel may also have an aspect ratio (length to average cross sectional dimension) of at least 2:1, more typically at least about 3:1, at least about 4:1, at least about 5:1, at least about 6:1, at least about 8:1, at least about 10:1, at least about 15:1, at least about 20:1, at least about 30:1, at least about 40:1, at least about 50:1, at least about 60:1, at least about 70:1, at least about 80:1, at least about 90:1, at least about 100:1 or more. An open channel generally will include characteristics that facilitate control over fluid transport, e.g., structural characteristics (an elongated indentation) and/or physical or chemical characteristics (hydrophobicity vs. hydrophilicity) or other characteristics that can exert a force (e.g., a containing force) on a fluid. Non-limiting examples of force actuators that can produce suitable forces include piezo actuators, pressure valves, electrodes to apply AC electric fields, and the like. The fluid within the channel may partially or completely fill the channel. In some cases where an open channel is used, the fluid may be held within the channel, for example, using surface tension (i.e., a concave or convex meniscus).

The channel may be of any size, for example, having a largest dimension perpendicular to net fluid flow of less than about 5 mm or 2 mm, or less than about 1 mm, less than about 500 microns, less than about 200 microns, less than about 100 microns, less than about 60 microns, less than about 50 microns, less than about 40 microns, less than about 30 microns, less than about 25 microns, less than about 10 microns, less than about 3 microns, less than about 1 micron, less than about 300 nm, less than about 100 nm, less than about 30 nm, or less than about 10 nm. In some cases, the dimensions of the channel are chosen such that fluid is able to freely flow through the device or substrate. The dimensions of the channel may also be chosen, for example, to allow a certain volumetric or linear flow rate of fluid in the channel. Of course, the number of channels and the shape of the channels can be varied by any method known to those of ordinary skill in the art. In some cases, more than one channel may be used. For example, two or more channels may be used, where they are positioned adjacent or proximate to each other, positioned to intersect with each other, etc.

In certain embodiments, one or more of the channels within the device may have an average cross-sectional dimension of less than about 10 cm. In certain instances, the average cross-sectional dimension of the channel is less than about 5 cm, less than about 3 cm, less than about 1 cm, less than about 5 mm, less than about 3 mm, less than about 1 mm, less than 500 micrometers, less than 200 micrometers, less than 100 micrometers, less than 50 micrometers, or less than 25 micrometers. The "average cross-sectional dimension" is measured in a plane perpendicular to net fluid flow within the channel. If the channel is non-circular, the average cross-sectional dimension may be taken as the diameter of a circle having the same area as the cross-sectional area of the channel. Thus, the channel may have any suitable cross-sectional shape, for example, circular, oval, triangular, irregular, square, rectangular, quadrilateral, or the like. In some embodiments, the channels are sized so as to allow laminar flow of one or more fluids contained within the channel to occur.

The channel may also have any suitable cross-sectional aspect ratio. The "cross-sectional aspect ratio" is, for the cross-sectional shape of a channel, the largest possible ratio (large to small) of two measurements made orthogonal to each other on the cross-sectional shape. For example, the channel may have a cross-sectional aspect ratio of less than about 2:1, less than about 1.5:1 , or in some cases about 1:1 (e.g., for a circular or a square cross-sectional shape). In other embodiments, the cross-sectional aspect ratio may be relatively large. For example, the cross-sectional aspect ratio may be at least about 2:1, at least about 3:1, at least about 4:1, at least about 5:1, at least about 6:1, at least about 7:1, at least about 8:1, at least about 10:1, at least about 12:1, at least about 15:1, or at least about 20:1.

As mentioned, the channels can be arranged in any suitable configuration within the device. Different channel arrangements may be used, for example, to manipulate fluids, droplets, and/or other species within the channels. For example, channels within the device can be arranged to create droplets (e.g., discrete droplets, single emulsions, double emulsions or other multiple emulsions, etc.), to mix fluids and/or droplets or other species contained therein, to screen or sort fluids and/or droplets or other species contained therein, to split or divide fluids and/or droplets, to cause a reaction to occur (e.g., between two fluids, between a species carried by a first fluid and a second fluid, or between two species carried by two fluids to occur), or the like.

Non-limiting examples of systems for manipulating fluids, droplets, and/or other species are discussed below. Additional examples of suitable manipulation systems can also be seen in U.S. Patent Application Serial No. 11/246,911, filed October 7, 2005, entitled "Formation and Control of Fluidic Species," by Link, *et al.,* published as U.S. Patent Application Publication No. 2006/0163385 on July 27, 2006; U.S. Patent Application Serial No. 11/024,228, filed December 28, 2004, entitled "Method and Apparatus for Fluid Dispersion," by Stone, *et al.,* now U.S. Patent No. 7,708,949, issued May 4, 2010; U.S. Patent Application Serial No. 11/885,306, filed August 29, 2007, entitled "Method and Apparatus for Forming Multiple Emulsions," by Weitz, *et al.,* published as U.S. Patent Application Publication No. 2009/0131543 on May 21, 2009; and U.S. Patent Application Serial No. 11/360,845, filed February 23, 2006, entitled "Electronic Control of Fluidic Species," by Link, *et al.,* published as U.S. Patent Application Publication No. 2007/0003442 on January 4, 2007.

Fluids may be delivered into channels within a device via one or more fluid sources. Any suitable source of fluid can be used, and in some cases, more than one source of fluid is used. For example, a pump, gravity, capillary action, surface tension, electroosmosis, centrifugal forces, etc. may be used to deliver a fluid from a fluid source into one or more channels in the device. A vacuum (e.g., from a vacuum pump or other suitable vacuum source) can also be used in some embodiments. Non-limiting examples of pumps include syringe pumps, peristaltic pumps, pressurized fluid sources, or the like. The device can have any number of fluid sources associated with it, for example, 1, 2, 3, 4, 5, 6, 7, 8, 9, 10, etc., or more fluid sources. The fluid sources need not be used to deliver fluid into the same channel, e.g., a first fluid source can deliver a first fluid to a first channel while a second fluid source can deliver a second fluid to a second channel, etc. In some cases, two or more channels are arranged to intersect at one or more intersections. There may be any number of fluidic channel intersections within the device, for example, 2, 3, 4, 5, 6, etc., or more intersections.

A variety of materials and methods, according to certain aspects of the disclosure, can be used to form devices or components such as those described herein, e.g., channels such as microfluidic channels, chambers, etc. For example, various devices or components can be formed from solid materials, in which the channels can be formed via micromachining, film deposition processes such as spin coating and chemical vapor deposition, physical vapor deposition, laser fabrication, photolithographic techniques, etching methods including wet chemical or plasma processes, electrodeposition, and the like. See, for example, Scientific American, 248:44-55, 1983 (Angell, *et al*)*.*

In one set of embodiments, various structures or components of the devices described herein can be formed of a polymer, for example, an elastomeric polymer such as polydimethylsiloxane ("PDMS"), polytetrafluoroethylene ("PTFE" or Teflon®), or the like. For instance, according to one embodiment, a channel such as a microfluidic channel may be implemented by fabricating the fluidic system separately using PDMS or other soft lithography techniques (details of soft lithography techniques suitable for this embodiment are discussed in the references entitled "Soft Lithography," by Younan Xia and George M. Whitesides, published in the Annual Review of Material Science, 1998, Vol. 28, pages 153-184, and "Soft Lithography in Biology and Biochemistry," by George M. Whitesides, Emanuele Ostuni, Shuichi Takayama, Xingyu Jiang and Donald E. Ingber, published in the Annual Review of Biomedical Engineering, 2001, Vol. 3, pages 335-373.

Other examples of potentially suitable polymers include, but are not limited to, polyethylene terephthalate (PET), polyacrylate, polymethacrylate, polycarbonate, polystyrene, polyethylene, polypropylene, polyvinylchloride, cyclic olefin copolymer (COC), polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The device may also be formed from composite materials, for example, a composite of a polymer and a semiconductor material.

In some embodiments, various structures or components of the device are fabricated from polymeric and/or flexible and/or elastomeric materials, and can be conveniently formed of a hardenable fluid, facilitating fabrication via molding (e.g. replica molding, injection molding, cast molding, etc.). The hardenable fluid can be essentially any fluid that can be induced to solidify, or that spontaneously solidifies, into a solid capable of containing and/or transporting fluids contemplated for use in and with the fluidic network. In one embodiment, the hardenable fluid comprises a polymeric liquid or a liquid polymeric precursor (i.e. a "prepolymer"). Suitable polymeric liquids can include, for example, thermoplastic polymers, thermoset polymers, waxes, metals, or mixtures or composites thereof heated above their melting point. As another example, a suitable polymeric liquid may include a solution of one or more polymers in a suitable solvent, which solution forms a solid polymeric material upon removal of the solvent, for example, by evaporation. Such polymeric materials, which can be solidified from, for example, a melt state or by solvent evaporation, are well known to those of ordinary skill in the art. A variety of polymeric materials, many of which are elastomeric, are suitable, and are also suitable for forming molds or mold masters, for embodiments where one or both of the mold masters is composed of an elastomeric material. A non-limiting list of examples of such polymers includes polymers of the general classes of silicone polymers, epoxy polymers, and acrylate polymers. Epoxy polymers are characterized by the presence of a three-membered cyclic ether group commonly referred to as an epoxy group, 1,2-epoxide, or oxirane. For example, diglycidyl ethers of bisphenol A can be used, in addition to compounds based on aromatic amine, triazine, and cycloaliphatic backbones. Another example includes the well-known Novolac polymers. Non-limiting examples of silicone elastomers suitable for use according to the invention include those formed from precursors including the chlorosilanes such as methylchlorosilanes, ethylchlorosilanes, phenylchlorosilanes, etc.

Silicone polymers are used in certain embodiments, for example, the silicone elastomer polydimethylsiloxane. Non-limiting examples of PDMS polymers include those sold under the trademark Sylgard by Dow Chemical Co., Midland, MI, and particularly Sylgard 182, Sylgard 184, and Sylgard 186. Silicone polymers including PDMS have several beneficial properties simplifying fabrication of various structures of the disclosure. For instance, such materials are inexpensive, readily available, and can be solidified from a prepolymeric liquid via curing with heat. For example, PDMSs are typically curable by exposure of the prepolymeric liquid to temperatures of about, for example, about 65 °C to about 75 °C for exposure times of, for example, at least about an hour. Also, silicone polymers, such as PDMS, can be elastomeric and thus may be useful for forming very small features with relatively high aspect ratios, necessary in certain embodiments of the invention. Flexible (e.g., elastomeric) molds or masters can be advantageous in this regard.

One advantage of forming structures such as microfluidic structures or channels from silicone polymers, such as PDMS, is the ability of such polymers to be oxidized, for example by exposure to an oxygen-containing plasma such as an air plasma, so that the oxidized structures contain, at their surface, chemical groups capable of cross-linking to other oxidized silicone polymer surfaces or to the oxidized surfaces of a variety of other polymeric and non-polymeric materials. Thus, structures can be fabricated and then oxidized and essentially irreversibly sealed to other silicone polymer surfaces, or to the surfaces of other substrates reactive with the oxidized silicone polymer surfaces, without the need for separate adhesives or other sealing means. In most cases, sealing can be completed simply by contacting an oxidized silicone surface to another surface without the need to apply auxiliary pressure to form the seal. That is, the pre-oxidized silicone surface acts as a contact adhesive against suitable mating surfaces. Specifically, in addition to being irreversibly sealable to itself, oxidized silicone such as oxidized PDMS can also be sealed irreversibly to a range of oxidized materials other than itself including, for example, glass, silicon, silicon oxide, quartz, silicon nitride, polyethylene, polystyrene, glassy carbon, and epoxy polymers, which have been oxidized in a similar fashion to the PDMS surface (for example, via exposure to an oxygen-containing plasma). Oxidation and sealing methods useful in the context of the present invention, as well as overall molding techniques, are described in the art, for example, in an article entitled "Rapid Prototyping of Microfluidic Systems and Polydimethylsiloxane," Anal. Chem., 70:474-480, 1998 (Duffy *et al.*).

Another advantage to forming channels or other structures (or interior, fluid-contacting surfaces) from oxidized silicone polymers is that these surfaces can be much more hydrophilic than the surfaces of typical elastomeric polymers (where a hydrophilic interior surface is desired). Such hydrophilic channel surfaces can thus be more easily filled and wetted with aqueous solutions than can structures comprised of typical, unoxidized elastomeric polymers or other hydrophobic materials.

In some aspects, such devices may be produced using more than one layer or substrate, e.g., more than one layer of PDMS. For instance, devices having channels with multiple heights and/or devices having interfaces positioned such as described herein may be produced using more than one layer or substrate, which may then be assembled or bonded together, e.g., e.g., using plasma bonding, to produce the final device. As a specific example, a device as discussed herein may be molded from masters comprising two or more layers of photoresists, e.g., where two PDMS molds are then bonded together by activating the PDMS surfaces using O₂ plasma or other suitable techniques. For example, in some cases, the masters from which the PDMS device is cast may contain one or multiple layers of photoresist, e.g., to form a 3D device. In some embodiments, one or more of the layers may have one or more mating protrusions and/or indentations which are aligned to properly align the layers, e.g., in a lock-and-key fashion. For example, a first layer may have a protrusion (having any suitable shape) and a second layer may have a corresponding indentation which can receive the protrusion, thereby causing the two layers to become properly aligned with respect to each other.

In some aspects, one or more walls or portions of a channel may be coated, e.g., with a coating material, including photoactive coating materials. For example, in some embodiments, each of the microfluidic channels at the common junction may have substantially the same hydrophobicity, although in other embodiments, various channels may have different hydrophobicities. For example a first channel (or set of channels) at a common junction may exhibit a first hydrophobicity, while the other channels may exhibit a second hydrophobicity different from the first hydrophobicity, e.g., exhibiting a hydrophobicity that is greater or less than the first hydrophobicity. Non-limiting examples of systems and methods for coating microfluidic channels, for example, with sol-gel coatings, may be seen in International Patent Application No. PCT/US2009/000850, filed February 11, 2009, entitled "Surfaces, Including Microfluidic Channels, With Controlled Wetting Properties," by Abate, *et al.,* published as WO 2009/120254 on October 1, 2009, and International Patent Application No. PCT/US2008/009477, filed August 7, 2008, entitled "Metal Oxide Coating on Surfaces," by Weitz, *et al.,* published as WO 2009/020633 on February 12, 2009. Other examples of coatings include polymers, metals, or ceramic coatings, e.g., using techniques known to those of ordinary skill in the art.

As mentioned, in some cases, some or all of the channels may be coated, or otherwise treated such that some or all of the channels, including the inlet and daughter channels, each have substantially the same hydrophilicity. The coating materials can be used in certain instances to control and/or alter the hydrophobicity of the wall of a channel. In some embodiments, a sol-gel is provided that can be formed as a coating on a substrate such as the wall of a channel such as a microfluidic channel. One or more portions of the sol-gel can be reacted to alter its hydrophobicity, in some cases. For example, a portion of the sol-gel may be exposed to light, such as ultraviolet light, which can be used to induce a chemical reaction in the sol-gel that alters its hydrophobicity. The sol-gel may include a photoinitiator which, upon exposure to light, produces radicals. Optionally, the photoinitiator is conjugated to a silane or other material within the sol-gel. The radicals so produced may be used to cause a condensation or polymerization reaction to occur on the surface of the sol-gel, thus altering the hydrophobicity of the surface. In some cases, various portions may be reacted or left unreacted, e.g., by controlling exposure to light (for instance, using a mask).

A variety of definitions are now provided which will aid in understanding various aspects of the invention. Following, and interspersed with these definitions, is further disclosure that will more fully describe the invention.

A "droplet," as used herein, is an isolated portion of a first fluid that is completely surrounded by a second fluid. In some cases, the first fluid and the second fluid are substantially immiscible. It is to be noted that a droplet is not necessarily spherical, but may assume other shapes as well, for example, depending on the external environment. The diameter of a droplet, in a non-spherical droplet, is the diameter of a perfect mathematical sphere having the same volume as the non-spherical droplet. The droplets may be created using any suitable technique, as previously discussed.

As used herein, a "fluid" is given its ordinary meaning, i.e., a liquid or a gas. A fluid cannot maintain a defined shape and will flow during an observable time frame to fill the container in which it is put. Thus, the fluid may have any suitable viscosity that permits flow. If two or more fluids are present, each fluid may be independently selected among essentially any fluids (liquids, gases, and the like) by those of ordinary skill in the art.

Certain embodiments of the present invention provide a plurality of droplets. In some embodiments, the plurality of droplets is formed from a first fluid, and may be substantially surrounded by a second fluid. As used herein, a droplet is "surrounded" by a fluid if a closed loop can be drawn around the droplet through only the fluid. A droplet is "completely surrounded" if closed loops going through only the fluid can be drawn around the droplet regardless of direction. A droplet is "substantially surrounded" if the loops going through only the fluid can be drawn around the droplet depending on the direction (e.g., in some cases, a loop around the droplet will comprise mostly of the fluid by may also comprise a second fluid, or a second droplet, etc.).

In most, but not all embodiments, the droplets and the fluid containing the droplets are substantially immiscible. In some cases, however, they may be miscible. In some cases, a hydrophilic liquid may be suspended in a hydrophobic liquid, a hydrophobic liquid may be suspended in a hydrophilic liquid, a gas bubble may be suspended in a liquid, etc. Typically, a hydrophobic liquid and a hydrophilic liquid are substantially immiscible with respect to each other, where the hydrophilic liquid has a greater affinity to water than does the hydrophobic liquid. Examples of hydrophilic liquids include, but are not limited to, water and other aqueous solutions comprising water, such as cell or biological media, ethanol, salt solutions, etc. Examples of hydrophobic liquids include, but are not limited to, oils such as ahydrocarbons, silicon oils, fluorocarbon oils, organic solvents etc. In some cases, two fluids can be selected to be substantially immiscible within the time frame of formation of a stream of fluids. Those of ordinary skill in the art can select suitable substantially miscible or substantially immiscible fluids, using contact angle measurements or the like, to carry out the techniques of the invention.

Reference is made to the following documents: International Patent Application No. PCT/US04/10903, filed April 9, 2004, entitled "Formation and Control of Fluidic Species," by Link, *et al.,* published as WO 2004/091763 on October 28, 2004; International Patent Application No. PCT/US03/20542, filed June 30, 2003, entitled "Method and Apparatus for Fluid Dispersion," by Stone, *et al.,* published as WO 2004/002627 on January 8, 2004; International Patent Application No. PCT/US04/27912, filed August 27, 2004, entitled "Electronic Control of Fluidic Species," by Link, *et al.,* published as WO 2005/021151 on March 10, 2005; and U.S. Pat. No. 8,337,778. Also provided as reference in their entireties are International Patent Application No. PCT/US2008/008563, filed July 11, 2008, entitled "Droplet-Based Selection," by Weitz, *et al.,* published as WO 2009/011808 on January 22, 2009; and International Patent Application No. PCT/US2009/004037, filed July 10, 2009, entitled "Systems and Methods of Droplet-Based Selection," by Weitz, *et al.,* published as WO 2010/005593 on July 10, 2009. WO 2015/031190 published on March 5, 2015 and claiming priority of U.S. Provisional Patent Application Serial No. 61/870,214, filed August 26, 2013, entitled "Determination of Immune Cells and Other Cells," is also provided as reference in its entirety.

The following examples are intended to illustrate certain embodiments of the present invention, but do not exemplify the full scope of the invention.

### EXAMPLE 1

The droplet-based assay in this example provides a high-throughput means to identify and recover individual T-cells that recognize specific target (e.g., infected or cancerous) cells. The method allows for the study of individual cells, can be rapid, can have very low reagent volumes, and/or can allow for selection and retrieval of individual cells that have a desired activity. After cells are retrieved, standard methods known to those of ordinary skill in the art can be used to obtain the sequence of the TCR for each retrieved cell of interest. For example, cell retrieval may be performed using methods of placing single droplets in individual reaction wells, e.g., as discussed in International Patent Application No. PCT/US 12/57404, filed September 27, 2012, entitled "Systems and Methods for Droplet Production and/or Fluidic Manipulation," by Sperling, *et al.,* published as WO2013/095737 on June 27, 2013.

The example described below shows a fundamentally new approach to examining the influence of one or several effector cells upon one or several target cells. This allows new applications involving on cell-to-cell signaling, cell killing mediated by direct cell-to-cell contact, killing of target cells via release of specific molecules from effector cells, etc. It should be noted that the different cell types do not need to be from the same patient or even from the same species. For example a single fungal cell could be co-encapsulated with bacterial cells to look for anti-bacterial compounds produced by the fungal cell. Or, fungal (or other) cells could be genetically modified to synthesize and secrete a library of peptides, one type of peptide from each cell. These cells could be encapsulated with bacterial cells to enable identification of fungal cells that produce library-encoded peptides with anti-bacterial activity.

Microfluidic water-in-oil droplets are produced by well-described methods, such as those described in International Patent Application No. PCT/US04/10903, filed April 9, 2004, entitled "Formation and Control of Fluidic Species," by Link, *et al.,* published as WO 2004/091763 on October 28, 2004, or International Patent Application No. PCT/US04/27912, filed August 27, 2004, entitled "Electronic Control of Fluidic Species," by Link, *et al.,* published as WO 2005/021151 on March 10, 2005. Cells of at least two different cell types may be included in the droplets. Generally, one cell type may be an effector, while the other cell type is a target cell which might respond to, or be influenced by, the effector cell. The effector cell can exert its influence through direct contact and/or indirectly. An example of a direct effect is a T-cell recognizing and killing its target cell. An example of an indirect effect is a cell that secretes a substance, e.g., a growth factor, that influences the target cell in a measurable way. In this case, the target cell might be genetically modified so that the growth factor induces expression of a marker protein, e.g., GFP, in the target cell.

This experiment uses model system cells to demonstrate that a killing assay can be performed in droplets. This type of killing assay could have many uses; for example, T-cells and tumor cells from a cancer patient and the T-cells capable of killing tumor cells could be isolated. T-cell recognition of target cells is governed by the specific type of T-cell receptor expressed on a given T-cell and as a result, isolation of T-cells with killing activity will enable identification of the T-cell receptor that recognizes the target cells.

This example illustrates a high-throughput method to assay the target-specific killing activity of individual patient-derived T-cells. The assay allows determination of: 1) the percent of total CD8+ T-cells with killing phenotype, 2) the number of unique CD8 killing clones, and 3) the frequency of each clone in the T-cell population.

Water-in-oil droplet microfluidics can be used to pair single effector and single target cells in 50 pL droplets. These droplets are produced at a high rate (e.g., greater than 1,000 droplets/s). The droplets are also stable during incubation, and suitable for cell culturing. The droplets can be observed optically and sorted at rates of, e.g., about 150 droplets/second to 500 droplets/second, or faster. After sorting, the droplets can be broken and the released cells can be diluted in wells for PCR amplification of individual T-cell receptor (TCR) genes. In this way, the TCR of each cell with killing activity can be identified, and histograms of TCR frequency can be generated.

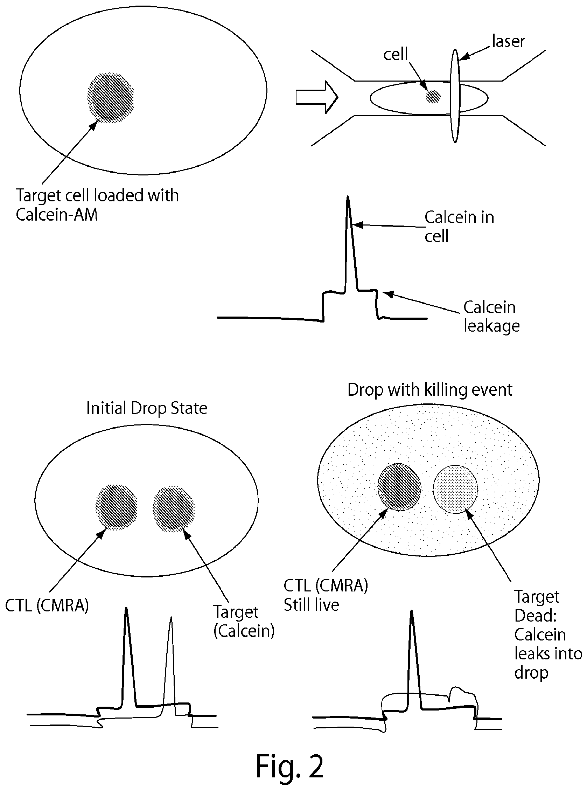

Co-flow microfluidics were used to create approximately 50 micrometer-diameter droplets containing a single effector cell and a single target cell. The target cells was labeled with Calcein AM (Invitrogen, Carlsbad, CA) and the effector cells was labeled with CMRA (Invitrogen). A killing event results in rapid loss of Calcein-AM from the target cell, while the effector cell retains its dye. The droplets can be monitored optically and sorted through microfluidic techniques. A schematic of the process is in Fig. 2.

Cells and cell culture: Effector NK92MI cells (part CRL-2408 and CCL-243, respectively, from ATCC, Manassas, VA) were cultured in Complete Alpha MEM: alpha-MEM (Invitrogen part 12571-048) supplemented with 0.1 mM 2-mercaptoethanol, 12% horse serum (ATCC Cat. No. 30-2040), and 12% fetal bovine calf serum (ATCC Cat. No. 30-2020). Target cells were K562 (ATCC) and cultured in RPMI supplemented with 10% FCS and 1% penicillin-streptomysin. Cells were split once every two or three days and kept at cell density between 50,000 and 400,000/ml.

Cell culturing and staining: K-562 target cells (part CCL-243, from ATCC, Manassas, VA) were cultured in RPMI 1640 (ATCC Cat. No. 30-2001) according to ATCC instructions. Prior to use in experiments, 10 ml cell culture (cell density -150,000 cells/ml) was removed to a 15 ml conical tube and 1 microliter 50 micromolar Calcein-AM in DMSO was added and mixed by inverting the capped tube. The cap was loosened and the cell/dye mixture was incubated 30 min in a 37 °C in cell incubator with 5% CO₂. After incubation, the cells were collected by centrifugation (6 minutes at 350x gravity) and washed twice with 2 ml Complete Alpha MEM. 400,000 cells were removed to an Eppendorf tube and reserved at room temp for 5 min prior to being combined with CMRA-stained NK92MI cells. Effector NK92MI cells (part CRL-2408 ATCC, Manassas, VA) were cultured in Complete Alpha MEM (a-MEM - Invitrogen part 12571-048, supplemented with 0.1 mM 2-mercaptoethanol, 12% horse serum - ATCC Cat. No. 30-2040, and 12% Fetal bovine calf serum - ATCC Cat. No. 30-2020) according to ATCC instructions. Prior to use in experiments, cells were stained with CMRA (Invitrogen part C34551 CellTracker™ Orange CMRA ex/em 548/ 576 nm) as follows. Lyophilized CMRA was dissolved in DMSO to a final concentration of 1 mM, and this stock solution was diluted to a final working concentration of 0.18 micromolar in serum-free medium. 5 ml cell culture (cell density -450,000 cells/ml) was removed to 15 ml conical tube and cells pelleted by centrifugation (4 minutes 250x gravity). The supernatant was completely removed and the cells were resuspended in 10 ml 37 °C alpha-MEM containing 0.18 micromolar final concentration CMRA. The cells were incubated for 20 min 37 °C, then collected by 4 minute centrifugation at 250x gravity. The supernatant was removed and cells were resuspended in 10 ml complete alpha-MEM warmed to 37 °C. After 15 minutes incubation at 37 °C, the cells were washed once with 2 ml, resuspended in 2 ml Complete Alpha-MEM, and the cell density determined. 400,000 cells were removed to an Eppendorf tube, centrifuged for 3 minutes at 250x gravity, and the supernatant was removed completely. The cells were resuspended in 100 microliters of a mixture containing 82 microliters of Complete Alpha MEM and 18 mircoliters Optiprep (Sigma Part No. D1556), used here as a density matching reagent. The resuspended cells were then used to resuspend the pellet of Calcein-AM-labeled K-562 target cells.

Microfluidic devices: Soft lithography was used to fabricate microfluidic channels in polydimethylsiloxane (PDMS, Sylgard 184 Silicone Elastomer, Dow Corning, Midland, MI). The desired design was printed onto a transparency (CAD/Art Services, Inc., Bandon, OR). SU8-3025 (Microchem, Newton, MA, USA) was spin-coated onto a cleaned silicon wafer to a final thickness of 25 mm following the manufacturer's protocol. Exposure to UV light (200-250 mJ, OAI, San Jose, CA) cross-linked the exposed pattern, and the non-exposed photoresist was removed using propylene glycol monomethyl ether acetate (PGMEA). PDMS with 10% (w/w) crosslinking agent was degassed and then poured onto the SU8-mold. After heating at 65 °C for more than 3 hours, the structure was peeled off the mold, plasma-treated, and bonded to a 25x75 mm glass slide of 1 mm thickness. Holes connecting to the channels were formed using biopsy punches (0.75 mm diameter Harris Uni-Core, Ted Pella, Inc., Redding, CA). Before use, channels are treated with Aquapel (PPG Industries, Pittsburgh, PA) followed by a flush with air to ensure that the oil carrier phase, and not the aqueous phase, wetted the surface.

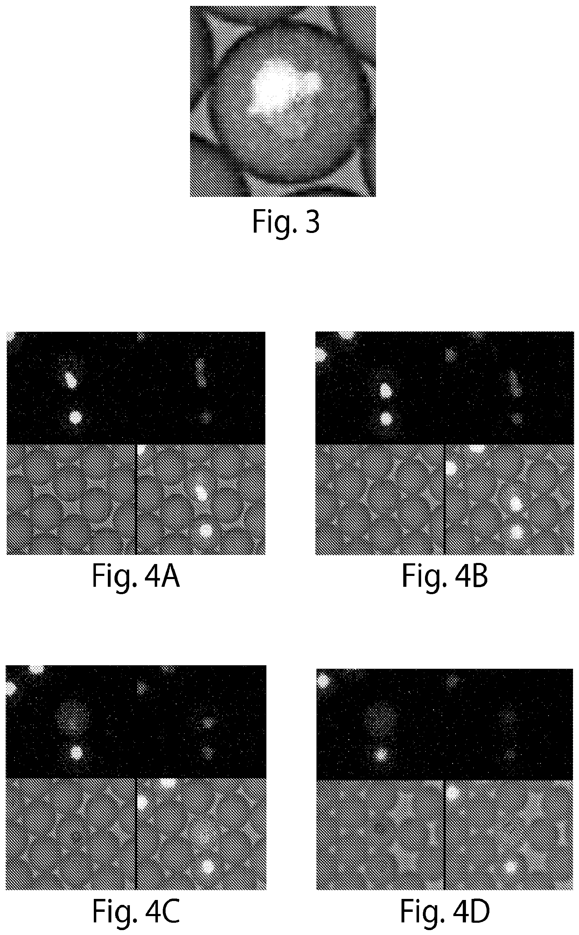

Cell encapsulation: 500 microliters HFE 7500 oil (without surfactant) was loaded into a 1 mL plastic syringe (Becton-Dickenson, Franklin Lakes, NJ, Part No. 309626). This syringe was held upright while the 100 microliters cell suspension was gently pipetted directly onto the oil in the syringe; simultaneously, the syringe plunger was slowly retracted to accommodate the sample. The oil in the syringe provided liquid to push the sample through the dead volume of the syringe and tubing, while the loading method prevented mixture of the oil and sample. The syringe was fitted with a 25G 5/8 gauge needle, polyethylene tubing (PE-20, Intramedic, Becton-Dickinson, Franklin Lakes, NJ) was inserted onto the needle, and the tubing was plugged into the one of the aqueous inlet holes on the PDMS device. A second 1 ml syringe, containing Complete Alpha-MEM, was attached to the second aqueous inlet. To encapsulate single cells in droplets, cell suspensions were flowed together with fluorinated oil (HFE7500, Sigma, St. Louis, MO) containing 1.8% (w/w) fluorinated surfactant using flow-focusing geometry to generate relatively monodisperse droplets of a water-in-oil emulsion. Syringe pumps (Harvard Apparatus, Holliston, MA) were used to control flow rates: 90 microliters/hr for each aqueous phase and 300 microliters/hr for the oil phase typically resulted in droplets of about 50 pL volume. The syringes were kept on ice throughout dropmaking and the droplets were collected through PE-20 tubing leading from the device exit into an Eppendorf kept on ice. To prevent droplet coalescence, the tubing outlet was under a layer of HFE7500 with surfactant. The aqueous droplets floated to the top of the much denser (1.6 g/cm³) oil to create a "creamed emulsion." Droplets were collected for 30 min. A droplet containing a single CMRA-stained NK92 effector cell in contact with a co-encapsulated Calcein-AM-stained K562 target cell is shown is Fig. 3.

Microscopy: Droplets were wicked into glass capillary tubes (VITROTUBES rectangular profile, 5 cm length, 0.5 mm width, 50 micrometer thickness, Cat. No. 5005-050 Fiber Optic Center Inc., New Bedford, MA), the tube ends closed were closed and affixed to microscope glass using vacuum grease. The droplets were imaged with a Leica TCS SP5 confocal microscope, using a 10x lens and 2.5x zoom. The microscope stage was heated to 37 °C. Each field was imaged at excitation/emission max of 488/520 (Calcein-AM) and 548/576 (CMRA), using bright field microscopy. Images were taken every minute over a two hour period. Images were converted to JPEG files and stacked into movie format using the Graphic Converter software package.

After droplet formation and collection on ice, the emulsion samples were wicked into glass capillary tubes and mounted on a confocal microscope stage heated to 37 °C. Droplets containing paired NK92MI and K-562 cells were located and these locations were imaged once per minute for two hours. In droplets containing either NK92MI or K-562 cells, the cells generally retained the dye for the duration of imaging. However, in droplets containing both a target and an effector cell, the target cell often released its dye contents suddenly, as seen by fluorescence imaging in the Calcein-AM channel. In these cases, bright-field imaging revealed that the target cell itself disappeared completely, strongly suggested that dye loss was the result of NK-mediated killing, rather than dye leakage. In contrast, the NK92MI effector cells retained the CMRA for long after the killing event, and any dye loss was due to gradual leakage (Fig. 4).

Figs. 4A-4D show that a NK92MI cell destroys co-encapsulated K-562 cell. Droplets in a 50 micrometer thick glass capillary were incubated at 37 °C for an indicated time. K-562 cells were labeled with Calcein-AM, 488 nm ex/521 nm em; NK92MI cells were labeled with CMRA, 548 nm ex/576 nm em. Note that the Calcein-AM signal bleeds into the 548/576 channel so that both cell types are seen. All cells retained dye after 60 minutes and 85 minutes incubation. Between minute 85 and minute 88, the K-562 cell encapsulated with an NK92MI cell suddenly released Calcein-AM. The co-encapsulated NK92MI cell retained CMRA. After 120 minutes incubation, CMRA dye is still retained by the co-encapsulated NK92MI cell. All singly-encapsulated cells retained dye throughout the entire 120-minute incubation.

Thus, this example demonstrates that droplet-based microfluidics can be the basis of a novel method to measure the effects of one or several effector cells on one or several target cells. This method can be combined with other experimental manipulations (both in-droplet and out-of-droplet) known to those of ordinary skill in the art. For example, droplets in which killing activity has occurred may be sorted and placed singly into microtiter wells, and the genes of interest within each cell may be amplified and sequenced.

### EXAMPLE 2

This example demonstrates a screen for cells that can produce neutralizing antibody. A schematic of this example is shown in Fig. 5A. In these examples, RAW 264.7 cells (3-5/drop) were infected with MNV-1 at ~1 PFU/drop, and RT-PCR was used to assess effect of A6.2 neutralizing antibody. Two sets of droplets were created: 1) virus, target cells, and A6.2 neutralizing hybridoma cells, and 2) virus, target cells, and irrelevant hybridoma cells. These were incubated to allow virus infection and replication. Then, the droplets were broken and RT-PCR was used to compare virus levels.

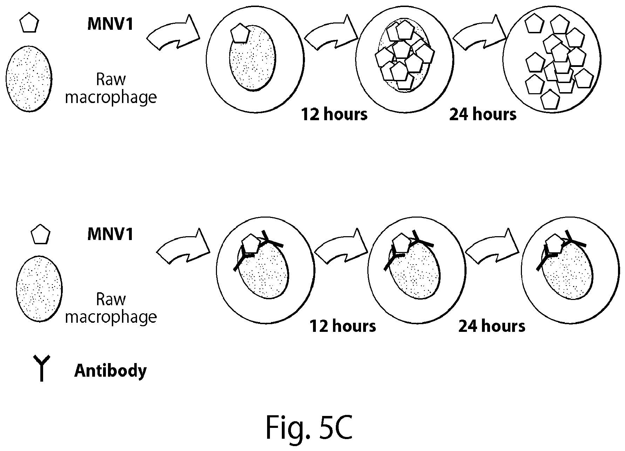

In Fig. 5C, in the presence of a neutralizing antibody (lower half of figure), the virus does not infect cells and does not replicate. Droplets that contain neutralizing antibodies thus have less viral RNA. As a result, RT-PCR on droplets containing virus and neutralizing antibody should generate less product than RT-PCR performed on droplets containing virus and a non-neutralizing (irrelevant) antibody.

Drop formation: Raw 264.7 target cells (~1 per drop) and purified norovirus (~1 PFU/drop) were encapsulated in 520 pL droplets along with either A6.2.1 hybridoma cells that secreted murine norovirus 1 (MNV1) neutralizing antibody, or with cells secreting an Irrelevant (non-neutralizing) anti-TNF alpha antibody. Secreting cells were included at ∼3 cells per drop. Droplets were prepared on ice and transferred to 37C for overnight incubation.

Real-time RT-PCR to measure effect of secreted antibodies: After incubation, the droplets were broken and Real-time RT-PCR (Real-time Reverse Transcriptase PCR) was used to quantify the amount of viral RNA in the lysed droplets. Control reactions were performed to ensure RT-PCR was free of contamination. Real time RT-PCR reactions were as follows: 1. "No Virus" RT-PCR samples contained reagents for reverse-transcriptase PCR of murine norovirus, but no viral template was included. 2. "RT-PCR + control" samples contained reagents for reverse-transcriptase PCR of murine norovirus, and viral template was included. 3. "Irrel. Hyb" samples contained reagents for reverse-transcriptase PCR of murine norovirus 1, and the template was a fraction of the aqueous phase of the broken droplets that had contained Raw 264.7 target cells, purified norovirus, and cells secreting an Irrelevant (non-neutralizing) anti-TNF alpha antibody. 4. "Neutr. Hyb A6.2" samples samples contained reagents for reverse-transcriptase PCR of murine norovirus, and the template was a fraction of the aqueous phase of the broken droplets that had contained Raw 264.7 target cells, purified norovirus, and A6.2.1 hybridoma cells that secrete Norovirus neutralizing antibody.

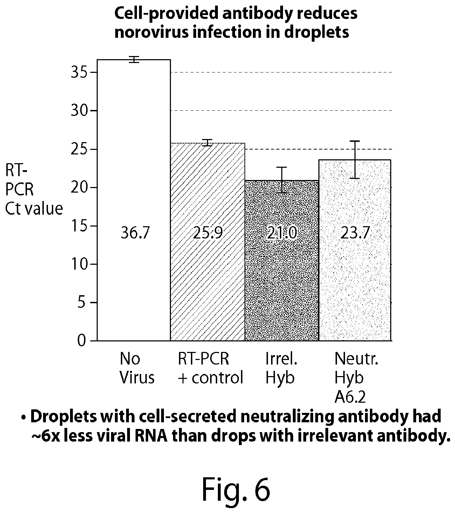

The RT-PCR was performed in triplicate and standard deviation error bars are shown in Fig. 6. Viral infection in the presence of cells that secreted neutralizing antibody resulted in increased Ct value relative to infection in the presence of cells secreting Irrelevant antibody, indicating that cells can provide neutralizing antibody for in-droplet viral neutralization assays.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A method of determining a sequence of an immune cell receptor, the method comprising: encapsulating immune cells and target cells in microfluidic droplets contained within a microfluidic channel such that at least some of the microfluidic droplets contain at least one immune cell and at least one target cell; determining viability of the target cell after exposure of the target cell to the immune cell; separating the microfluidic droplets on the basis of the viability of the target cell by determining a signaling entity within the droplets, wherein the signaling entity is leaked from the target cell containing therein said signaling entity; and for the microfluidic droplets containing at least one immune cell and at least one non-viable target cell, determining a receptor sequence of the at least one immune cell.

2. The method of claim 1, wherein the immune cells comprise:
(a) T-cells, preferably CD8+ T-cells; and/or
(b) B-cells; and/or
(c) cancer cells; and/or
(d) virally-infected cells.

3. The method of claim 1 or claim 2, wherein the immune cells and the target cells arise from the same organism or different organisms.

4. The method of any one of claims 1-3, wherein the immune cells are human.

5. The method of any one of claims 1-4, wherein the microfluidic droplets have a distribution in diameters such that no more than 5% of the droplets have a diameter greater than about 110% and/or less than about 90% of the overall average cross-sectional dimension of the droplets.

6. The method of any one of claims 1-5, wherein the microfluidic channel has an average cross-sectional dimension of less than about 1 mm.

7. The method of any one of claims 1-6, wherein at least about 80% of the microfluidic droplets contain at least one immune cell and at least one target cell, preferably wherein at least about 95% of the microfluidic droplets contain at least one immune cell and at least one target cell.

8. The method of any one of claims 1-7, wherein the signaling entity is fluorescent and/or wherein the signaling entity is calcein and/or a calcein derivative.

9. The method of claim 8, further comprising inserting the signaling entity into at least some of the target cells.

10. The method of any one of claims 1-9, wherein in at least some of the microfluidic droplets, the immune cell directly kills the target cell by phagocytosis.

11. The method of any one of claims 1-10, wherein in at least some of the microfluidic droplets, the immune cell kills the target cell by secreting a substance that kills the target cell, preferably wherein the substance is a cytolytic protein, preferably wherein the cytolitc protein is perforin, and/or preferably wherein the substance comprises a granzyme.

12. The method of any one of claims 1-11, wherein determining a receptor sequence comprises sequencing at least a portion of the DNA within the immune cells, preferably using PCR.

13. The method of any one of claims 1-12, comprising encapsulating immune cells and target cells at a rate of at least 1 droplet/s.

14. The method of any one of claims 1-13, further comprising culturing cells from at least some of the microfluidic droplets containing at least one immune cell and at least one non-viable target cell.

## Patentansprüche

1. Verfahren zum Bestimmen einer Sequenz eines Immunzellrezeptors, wobei das Verfahren umfasst: Einkapseln von Immunzellen und Zielzellen in mikrofluidischen Tröpfchen, die innerhalb eines mikrofluidischen Kanals enthalten sind, so dass wenigstens einige der mikrofluidischen Tröpfchen wenigstens eine Immunzelle und wenigstens eine Zielzelle enthalten; Bestimmen der Lebensfähigkeit der Zielzelle nach Aussetzen der Zielzelle an die Immunzelle; Trennen der mikrofluidischen Tröpfchen auf Grundlage der Lebensfähigkeit der Zielzelle durch Bestimmen einer Signalgebungseinheit innerhalb der Tröpfchen, wobei die Signalgebungseinheit aus der Zielzelle, welche darin die Signalgebungseinheit enthält, aussickert; und für die mikrofluidischen Tröpfchen, die wenigstens eine Immunzelle und wenigstens eine nicht-lebensfähige Zielzelle enthalten, Bestimmen einer Rezeptorsequenz der wenigstens einen Immunzelle.

2. Verfahren gemäß Anspruch 1, wobei die Immunzellen umfassen:
(a) T-Zellen, bevorzugt CD8+-T-Zellen; und/oder
(b) B-Zellen; und/oder
(c) Krebszellen; und/oder
(d) virusinfizierte Zellen.

3. Verfahren gemäß Anspruch 1 oder Anspruch 2, wobei die Immunzellen und die Zielzellen aus dem gleichen Organismen oder unterschiedlichen Organismen stammen.

4. Verfahren gemäß irgendeinem der Ansprüche 1-3, wobei die Immunzellen human sind.

5. Verfahren gemäß irgendeinem der Ansprüche 1-4, wobei die mikrofluidischen Tröpfchen eine solche Durchmesserverteilung haben, dass nicht mehr als 5% der Tröpfchen einen Durchmesser von mehr als etwa 110% und/oder weniger als etwa 90% der gesamten durchschnittlichen Querschnittsdimension der Tröpfchen haben.

6. Verfahren gemäß irgendeinem der Ansprüche 1-5, wobei der mikrofluidische Kanal eine durchschnittliche Querschnittsdimension von weniger als etwa 1 mm hat.

7. Verfahren gemäß irgendeinem der Ansprüche 1-6, wobei wenigstens etwa 80% der mikrofluidischen Tröpfchen wenigstens eine Immunzelle und wenigstens eine Zielzelle enthalten, wobei bevorzugt wenigstens etwa 95% der mikrofluidischen Tröpfchen wenigstens eine Immunzelle und wenigstens eine Zielzelle enthalten.

8. Verfahren gemäß irgendeinem der Ansprüche 1-7, wobei die Signalgebungseinheit fluoreszierend ist und/oder wobei die Signalgebungseinheit Calcein und/oder ein Calceinderivat ist.

9. Verfahren gemäß Anspruch 8, welches weiterhin das Einsetzen der Signalgebungseinheit in wenigstens einige der Zielzellen umfasst.

10. Verfahren gemäß irgendeinem der Ansprüche 1-9, wobei in wenigstens einigen der mikrofluidischen Tröpfchen die Immunzelle die Zielzelle durch Phagozytose direkt abtötet.

11. Verfahren gemäß irgendeinem der Ansprüche 1-10, wobei in wenigstens einigen der mikrofluidischen Tröpfchen die Immunzelle die Zielzelle durch Sekretieren einer Substanz abtötet, welche die Zielzelle abtötet, wobei bevorzugt die Substanz ein zytolytisches Protein ist, wobei bevorzugt das zytolytische Protein Perforin ist und/oder wobei bevorzugt die Substanz ein Granzyme umfasst.

12. Verfahren gemäß irgendeinem der Ansprüche 1-11, wobei das Bestimmen einer Rezeptorsequenz das Sequenzieren wenigstens eines Teils der DNA innerhalb der Immunzellen, bevorzugt unter Verwendung von PCR, umfasst.

13. Verfahren gemäß irgendeinem der Ansprüche 1-12, welches das Einkapseln von Immunzellen und Zielzellen bei einer Rate von wenigstens 1 Tröpfchen/s umfasst.

14. Verfahren gemäß irgendeinem der Ansprüche 1-13, welches weiterhin das Kultivieren von Zellen aus wenigstens einigen der mikrofluidischen Tröpfchen, die wenigstens eine Immunzelle und wenigstens eine nicht-lebensfähige Zielzelle enthalten, umfasst.

## Revendications

1. Méthode de détermination d'une séquence d'un récepteur de cellule immunitaire, la méthode comprenant :
l'encapsulation de cellules immunitaires et de cellules cibles dans des gouttelettes microfluidiques contenues à l'intérieur d'un canal microfluidique de telle sorte qu'au moins certaines des gouttelettes microfluidiques contiennent au moins une cellule immunitaire et au moins une cellule cible ;
la détermination de la viabilité de la cellule cible après l'exposition de la cellule cible à la cellule immunitaire ;
la séparation des gouttelettes microfluidiques sur la base de la viabilité de la cellule cible en déterminant une entité de signalisation à l'intérieur des gouttelettes,
dans laquelle l'entité de signalisation découle de la cellule cible contenant dans celle-ci ladite entité de signalisation ; et pour les gouttelettes microfluidiques contenant au moins une cellule immunitaire et au moins une cellule cible non viable, la détermination d'une séquence de récepteur de l'au moins une cellule immunitaire.

2. La méthode de la revendication 1, dans laquelle les cellules immunitaires comprennent :
(a) des cellules T, préférablement des cellules T CD8+ ; et/ou
(b) des cellules B ; et/ou
(c) des cellules cancéreuses ; et/ou
(d) des cellules infectées par un virus.

3. La méthode de la revendication 1 ou de la revendication 2, dans laquelle les cellules immunitaires et les cellules cibles proviennent du même organisme ou d'organismes différents.

4. La méthode de l'une quelconque des revendications 1-3, dans laquelle les cellules immunitaires sont humaines.

5. La méthode de l'une quelconque des revendications 1-4, dans laquelle les gouttelettes microfluidiques ont une distribution en diamètres de telle sorte que pas plus que 5% des gouttelettes ont un diamètre supérieur d'environ 110% et/ou inférieur d'environ 90% de la dimension transversale moyenne entière des gouttelettes.

6. La méthode de l'une quelconque des revendications 1-5, dans laquelle le canal microfluidique a une dimension transversale moyenne inférieure à environ 1 mm.

7. La méthode de l'une quelconque des revendications 1-6, dans laquelle au moins 80% des gouttelettes microfluidiques contiennent au moins une cellule immunitaire et au moins une cellule cible, préférablement dans laquelle au moins 95% des gouttelettes microfluidiques contiennent au moins une cellule immunitaire et au moins une cellule cible.

8. La méthode de l'une quelconque des revendications 1-7, dans laquelle l'entité de signalisation est fluorescente et/ou dans laquelle l'entité de signalisation est la calcéine et/ou un dérivé de calcéine.

9. La méthode de la revendication 8, comprenant en outre l'insertion de l'entité de signalisation dans au moins certaines de cellules cibles.

10. La méthode de l'une quelconque des revendications 1-9, dans laquelle dans au moins certaines des gouttelettes microfluidiques, la cellule immunitaire tue directement la cellule cible par phagocytose.

11. La méthode de l'une quelconque des revendications 1-10, dans laquelle dans au moins certaines des gouttelettes microfluidiques, la cellule immunitaire tue la cellule cible en sécrétant une substance qui tue la cellule cible, préférablement dans laquelle la substance est une protéine cytolytique, préférablement dans laquelle la protéine cytolytique est la perforine, et/ou préférablement dans laquelle la substance comprend une granzyme.

12. La méthode de l'une quelconque des revendications 1-11, dans laquelle la détermination d'une séquence de récepteur comprend le séquençage d'au moins une portion de l'ADN à l'intérieur des cellules immunitaires, préférablement en utilisant polymérase en temps réel (PCR).

13. La méthode de l'une quelconque des revendications 1-12, comprenant l'encapsulation des cellules immunitaires et des cellules cibles à une vitesse d'au moins une gouttelette/s.

14. La méthode de l'une quelconque des revendications 1-13, comprenant en outre la culture de cellules d'au moins certaines des gouttelettes microfluidiques contenant au moins une cellule immunitaire et au moins une cellule cible non viable.
